(19) Europäisches Patentamt
European Patent Office
Office européen des brevets

(11) EP 4 273 138 A1

(12) **EUROPEAN PATENT APPLICATION**
published in accordance with Art. 153(4) EPC

(43) Date of publication:
**08.11.2023 Bulletin 2023/45**

(21) Application number: **21914638.8**

(22) Date of filing: **30.12.2021**

(51) International Patent Classification (IPC):
***C07D 401/10*** *(2006.01)* ***A61K 31/4439*** *(2006.01)*
***A61P 35/00*** *(2006.01)*

(52) Cooperative Patent Classification (CPC):
**A61K 31/4439; A61P 35/00; C07D 401/10**

(86) International application number:
**PCT/CN2021/143129**

(87) International publication number:
**WO 2022/143907 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.12.2020 CN 202011613790**

(71) Applicant: **CHIA TAI TIANQING PHARMACEUTICAL GROUP CO., LTD.**
**Lianyungang,**
**Jiangsu 222062 (CN)**

(72) Inventors:
- **DUAN, Shuwen**
  **Shanghai 200131 (CN)**
- **LU, Jianyu**
  **Shanghai 200131 (CN)**
- **YAO, Ting**
  **Shanghai 200131 (CN)**
- **HU, Lihong**
  **Shanghai 200131 (CN)**
- **DING, Charles Z.**
  **Shanghai 200131 (CN)**
- **LI, Jian**
  **Shanghai 200131 (CN)**
- **CHEN, Shuhui**
  **Shanghai 200131 (CN)**
- **ZHANG, Xiquan**
  **Lianyungang, Jiangsu 222062 (CN)**

(74) Representative: **Grünecker Patent- und Rechtsanwälte**
**PartG mbB**
**Leopoldstraße 4**
**80802 München (DE)**

(54) **SALT FORM OF TETRA-SUBSTITUTED OLEFIN COMPOUND, CRYSTAL, AND PREPARATION METHOD THEREFOR**

(57) A salt form of a tetra-substituted olefin compound, a crystal of the compound and the salt, and a preparation method therefor. Specifically, the present invention relates to a crystal of a compound represented by formula (I), a salt thereof, a crystal of the salt thereof, and a preparation method therefor.

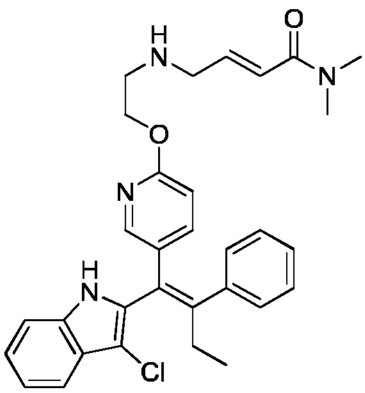

(I)

EP 4 273 138 A1

## Description

### CROSS-REFERENCE TO RELATED APPLICATIONS

The present application claims the benefit and priority to the Chinese Patent Application No. 202011613790.2 filed with National Intellectual Property Administration, PRC on December 30, 2020, which is incorporated herein by reference in its entirety.

### TECHNICAL FIELD

The present application relates to a crystalline form of a tetra-substituted olefin compound, a salt thereof, a crystalline form of the salt thereof, and a preparation method therefor.

### BACKGROUND

According to WHO statistics, breast cancer ranks the second among cancers in global population and the first in women in incidence. After years of research, the role of the estrogen-estrogen receptor signaling pathway in the development of breast cancer has been determined; estrogen receptor (ER) has become the most important biomarker of breast cancer. Based on estrogen receptor expression, breast cancers can be classified into estrogen receptor positive breast cancer and estrogen receptor negative breast cancer. Among them, estrogen receptor positive breast cancer accounts for more than 70% in breast cancer patients.

Endocrine therapy (ET) for the estrogen-estrogen receptor signaling pathway in breast cancer cells has become the first choice for estrogen receptor positive breast cancer due to its minimal harm and remarkable efficacy. Generally, the first-line endocrine therapy is aromatase inhibitors (AIs). Although the aromatase inhibitor letrozole has demonstrated good efficacy in treating estrogen receptor positive breast cancer, with the application of AIs, the resistance problem of estrogen receptor positive breast cancer to AIs is becoming prominent. A large number of studies suggest that for AIs, the estrogen receptor gene may mutate, mainly in Y537X, producing a estrogen receptor mutant that may keep an excited conformation in the absence of estrogen and continue to function as a receptor to promote breast cancer cell proliferation. As the only marketed selective estrogen receptor down-regulator, fulvestrant has demonstrated good results in treating hormone-resistant breast cancer. However, fulvestrant has many problems with the treatment of AI-resistant ER mutant breast cancer. Due to its poor pharmacokinetics (PK), fulvestrant shows zero bioavailability via oral administration, while having a high blood clearance rate. For the above two reasons, this drug can only be administered by intramuscular injection. However, because of its strong lipophilicity, fulvestrant administered by intramuscular injection also has serious problems in tissue distribution, resulting in a clinical response rate of about 50% in breast cancer patients. Also due to the poor PK properties, the current approved dosage of fulvestrant cannot cause complete degradation of ER, especially mutant ER, at tissue concentration. Therefore, the therapy is far from optimal for AI-resistant ER-mutant breast cancer. Therefore, the development of medications targeting ER-mutant breast cancer with better PK properties remains an unmet medical need.

### BRIEF SUMMARY

In one aspect, the present application provides a crystalline form of a compound of formula (I),

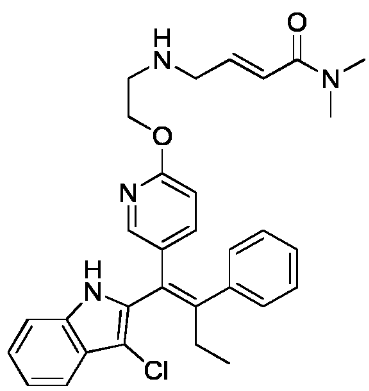

(I)

In some embodiments of the present application, the crystalline form of the compound of formula (I) has diffraction peaks in an X-ray powder diffraction pattern using Cu Kα radiation at 2Θ values of 17.60±0.20°, 19.98±0.20°, and 23.41±0.20°.

In some embodiments of the present application, the crystalline form of the compound of formula (I) has diffraction peaks in an X-ray powder diffraction pattern using Cu Kα radiation at 2Θ values of 13.14±0.20°, 16.62±0.20°, 17.60±0.20°, 19.98±0.20°, 21.78±0.20°, 22.38±0.20°, 23.41±0.20°, and 24.22±0.20°.

In some embodiments of the present application, the crystalline form of the compound of formula (I) has diffraction peaks in an X-ray powder diffraction pattern using Cu Kα radiation at 2Θ values of 13.14±0.20°, 14.68±0.20°, 16.62±0.20°, 17.60±0.20°, 19.98±0.20°, 21.32±0.20°, 21.78±0.20°, 22.38±0.20°, 23.41±0.20°, 24.22±0.20°, 26.46±0.20°, and 28.84±0.20°.

In some embodiments of the present application, the crystalline form of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 8.56±0.20°, 10.13±0.20°, 12.16±0.20°, 13.14±0.20°, 13.54±0.20°, 14.68±0.20°, 15.68±0.20°, 16.38±0.20°, 16.62±0.20°, 17.24±0.20°, 17.60±0.20°, 18.86±0.20°, 19.22±0.20°, 19.46±0.20°, 19.98±0.20°, 20.89±0.20°, 21.32±0.20°, 21.78±0.20°, 22.38±0.20°, 22.70±0.20°, 23.08±0.20°, 23.41±0.20°, 23.70±0.20°, 24.01±0.20°, 24.22±0.20°, 24.62±0.20°, 24.89±0.20°, 25.26±0.20°, 25.92±0.20°, 26.46±0.20°, 26.92±0.20°, 27.32±0.20°, 28.18±0.20°, 28.54±0.20°, 28.84±0.20°, 29.42±0.20°, 30.24±0.20°, 30.70±0.20°, 30.94±0.20°, 31.34±0.20°, 31.64±0.20°, 32.71±0.20°, 33.22±0.20°, and 34.84±0.20°.

In some embodiments of the present application, the crystalline form of the compound of formula (I) comprises 3, 4, 5, 6, 7, 8, 9, 10, 11, or 12 diffraction peaks in an X-ray powder diffraction pattern using Cu Kα radiation at 2Θ values selected from the group consisting of: 13.14±0.20°, 14.68±0.20°, 16.62±0.20°, 17.60±0.20°, 19.98±0.20°, 21.32±0.20°, 21.78±0.20°, 22.38±0.20°, 23.41±0.20°, 24.22±0.20°, 26.46±0.20°, and 28.84±0.20°.

In some embodiments of the present application, the crystalline form of the compound of formula (I) comprises 3, 4, 5, 6, 7, or 8 diffraction peaks in an X-ray powder diffraction pattern using Cu Kα radiation at 2Θ values selected from the group consisting of: 13.14±0.20°, 16.62±0.20°, 17.60±0.20°, 19.98±0.20°, 21.78±0.20°, 22.38±0.20°, 23.41±0.20°, and 24.22±0.20°.

In some embodiments of the present application, the crystalline form of the compound of formula (I) has diffraction peaks in an X-ray powder diffraction pattern using Cu Kα radiation at 2Θ values of about 8.56°, 10.13°, 12.16°, 13.14°, 13.54°, 14.68°, 15.68°, 16.38°, 16.62°, 17.24°, 17.60°, 18.86°, 19.22°, 19.46°, 19.98°, 20.89°, 21.32°, 21.78°, 22.38°, 22.70°, 23.08°, 23.41°, 23.70°, 24.01°, 24.22°, 24.62°, 24.89°, 25.26°, 25.92°, 26.46°, 26.92°, 27.32°, 28.18°, 28.54°, 28.84°, 29.42°, 30.24°, 30.70°, 30.94°, 31.34°, 31.64°, 32.71°, 33.22°, and 34.84°.

In some embodiments of the present application, in an X-ray powder diffraction pattern of the crystalline form of the compound of formula (I) using Cu Kα radiation, the peak positions and relative intensities of diffraction peaks are shown in Table 1 below:

**Table 1. Peak positions and relative intensities of diffraction peaks in the X-ray powder diffraction pattern of the crystalline form of the compound of formula (I)**

| No. | 2θ[°]±0.2° | Relative intensity [%] | No. | 2θ[°]±0.2° | Relative intensity [%] |
|---|---|---|---|---|---|
| 1 | 8.56 | 3.9 | 23 | 23.7 | 22.5 |
| 2 | 10.13 | 7.2 | 24 | 24.01 | 6.7 |
| 3 | 12.16 | 8.9 | 25 | 24.22 | 23.9 |
| 4 | 13.14 | 28.8 | 26 | 24.62 | 14.7 |
| 5 | 13.54 | 43 | 27 | 24.89 | 3 |
| 6 | 14.68 | 20.2 | 28 | 25.26 | 12.7 |
| 7 | 15.68 | 7.4 | 29 | 25.92 | 7.5 |
| 8 | 16.38 | 19.5 | 30 | 26.46 | 21.8 |
| 9 | 16.62 | 22.3 | 31 | 26.92 | 8.2 |
| 10 | 17.24 | 3.1 | 32 | 27.32 | 3.9 |
| 11 | 17.60 | 79.7 | 33 | 28.18 | 14.3 |
| 12 | 18.86 | 7.8 | 34 | 28.54 | 12 |

(continued)

| No. | 2θ[°]±0.2° | Relative intensity [%] | No. | 2θ[°]±0.2° | Relative intensity [%] |
|---|---|---|---|---|---|
| 13 | 19.22 | 4.8 | 35 | 28.84 | 22.2 |
| 14 | 19.46 | 2.8 | 36 | 29.42 | 5.8 |
| 15 | 19.98 | 100 | 37 | 30.24 | 4 |
| 16 | 20.89 | 3.1 | 38 | 30.70 | 11.6 |
| 17 | 21.32 | 20.3 | 39 | 30.94 | 8.8 |
| 18 | 21.78 | 37.3 | 40 | 31.64 | 6.4 |
| 19 | 22.38 | 49 | 41 | 32.71 | 3.3 |
| 20 | 22.70 | 3.3 | 42 | 33.22 | 10.4 |
| 21 | 23.08 | 9.7 | 43 | 34.84 | 5.2 |
| 22 | 23.41 | 64.3 | / | / | / |

In some embodiments of the present application, the crystalline form of the compound of formula (I) has an X-ray powder diffraction (XRPD) pattern using Cu Kα radiation as shown in FIG. 1.

In some embodiments of the present application, the crystalline form of the compound of formula (I) has a differential scanning calorimetry (DSC) curve showing an endothermic peak at 144.92±3 °C.

In some embodiments of the present application, the crystalline form of the compound of formula (I) has a DSC pattern as shown in FIG. 2.

In some embodiments of the present application, the crystalline form of the compound of formula (I) has a thermogravimetric analysis (TGA) curve showing a weight loss of 0.108% at 200.00±3 °C.

In some embodiments of the present application, the crystalline form of the compound of formula (I) has a TGA pattern as shown in FIG. 3.

In another aspect, the present application provides a method for preparing the crystalline form of the compound of formula (I), comprising a step of precipitating the crystalline form of the compound of formula (I) in a solvent of ethyl acetate.

In some embodiments of the present application, the present application provides a method for preparing the crystalline form of the compound of formula (I), comprising: adding ethyl acetate to the compound of formula (I), heating and stirring until dissolved, cooling and stirring, and after crystallization and precipitation, filtering and drying under reduced pressure to give the crystalline form of the compound of formula (I).

In another aspect, the present application further provides a crystalline form I of a hydrochloride of the compound of formula (I).

In some embodiments of the present application, the crystalline form I of the hydrochloride of the compound of formula (I) has diffraction peaks in an X-ray powder diffraction pattern using Cu Kα radiation at 2Θ values of 5.97±0.20°, 7.58±0.20°, and 17.63±0.20°.

In some embodiments of the present application, the crystalline form I of the hydrochloride of the compound of formula (I) has diffraction peaks in an X-ray powder diffraction pattern using Cu Kα radiation at 2Θ values of 5.97±0.20°, 7.58±0.20°, 16.49±0.20°, 17.63±0.20°, 20.25±0.20°, 22.84±0.20°, 23.99±0.20°, and 24.62±0.20°.

In some embodiments of the present application, the crystalline form I of the hydrochloride of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 5.97±0.20°, 7.58±0.20°, 11.08±0.20°, 11.38±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.99±0.20°, and 24.62±0.20°.

In some embodiments of the present application, the crystalline form I of the hydrochloride of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 5.97±0.20°, 7.58±0.20°, 9.83±0.20°, 11.08±0.20°, 11.38±0.20°, 12.69±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.79±0.20°, 23.99±0.20°, and 24.62±0.20°.

In some embodiments of the present application, the crystalline form I of the hydrochloride of the compound of formula (I) comprises 3, 4, 5, 6, 7, or 8 diffraction peaks in an X-ray powder diffraction pattern using Cu Kα radiation

at 2Θ values selected from the group consisting of: 5.97±0.20°, 7.58±0.20°, 16.49±0.20°, 17.63±0.20°, 20.25±0.20°, 22.84±0.20°, 23.99±0.20°, and 24.62±0.20°.

In some embodiments of the present application, the crystalline form I of the hydrochloride of the compound of formula (I) has diffraction peaks in an X-ray powder diffraction pattern using Cu Kα radiation at 2Θ values of 5.97±0.20°, 7.58±0.20°, 11.08±0.20°, 11.38±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.99±0.20°, and 24.62±0.20°.

In some embodiments of the present application, the crystalline form I of the hydrochloride of the compound of formula (I) has diffraction peaks in an X-ray powder diffraction pattern using Cu Kα radiation at 2Θ values of 5.97±0.20°, 7.58±0.20°, 9.83±0.20°, 11.08±0.20°, 11.38±0.20°, 12.69±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.79±0.20°, 23.99±0.20°, and 24.62±0.20°.

In some embodiments of the present application, the crystalline form I of the hydrochloride of the compound of formula (I) has diffraction peaks in an X-ray powder diffraction pattern using Cu Kα radiation at 2Θ values of about 5.97°, 7.58°, 11.08°, 11.38°, 14.14°, 15.30°, 16.49°, 17.63°, 18.44°, 19.72°, 20.25°, 20.66°, 21.39°, 21.88°, 22.23°, 22.84°, 23.99°, 24.62°, 25.15°, 25.74°, 26.19°, 27.03°, 27.43°, 27.88°, 28.52°, 29.08°, 30.08°, 30.47°, 31.51°, and 31.94°.

In some embodiments of the present application, the crystalline form I of the hydrochloride of the compound of formula (I) has diffraction peaks in an X-ray powder diffraction pattern using Cu Kα radiation at 2Θ values of about 5.97°, 7.58°, 9.83°, 11.08°, 11.38°, 12.69°, 14.14°, 15.30°, 16.49°, 17.63°, 18.44°, 19.72°, 20.25°, 20.66°, 21.39°, 21.88°, 22.23°, 22.84°, 23.79°, 23.99°, 24.62°, 25.15°, 25.74°, 26.19°, 27.03°, 27.43°, 27.88°, 28.52°, 29.08°, 30.08°, 30.47°, 31.51°, and 31.94°.

In some embodiments of the present application, in an X-ray powder diffraction pattern of the crystalline form I of the hydrochloride of the compound of formula (I) using Cu Kα radiation, the peak positions and relative intensities of diffraction peaks are shown in Table 2 below:

**Table 2. Peak positions and relative intensities of diffraction peaks in the X-ray powder diffraction pattern of the crystalline form I of the hydrochloride of the compound of formula (I)**

| No. | 2θ[°]±0.20° | Relative intensity [%] | No. | 2θ[°]±0.20° | Relative intensity [%] |
|---|---|---|---|---|---|
| 1 | 5.97 | 73.45 | 18 | 22.84 | 30.58 |
| 2 | 7.58 | 88.58 | 19 | 23.79 | 35.87 |
| 3 | 9.83 | 4.85 | 20 | 23.99 | 69.14 |
| 4 | 11.08 | 7.62 | 21 | 24.62 | 37.48 |
| 5 | 11.38 | 8.75 | 22 | 25.15 | 6.13 |
| 6 | 12.69 | 2.46 | 23 | 25.74 | 3.86 |
| 7 | 14.14 | 3.22 | 24 | 26.19 | 13.5 |
| 8 | 15.30 | 9.31 | 25 | 27.03 | 9.7 |
| 9 | 16.49 | 34.34 | 26 | 27.43 | 11.37 |
| 10 | 17.63 | 100 | 27 | 27.88 | 14.57 |
| 11 | 18.44 | 21.8 | 28 | 28.52 | 6.91 |
| 12 | 19.72 | 14.89 | 29 | 29.08 | 8.17 |
| 13 | 20.25 | 29.98 | 30 | 30.08 | 8.43 |
| 14 | 20.66 | 19.91 | 31 | 30.47 | 13.95 |
| 15 | 21.39 | 11.41 | 32 | 31.51 | 8.49 |
| 16 | 21.88 | 6.17 | 33 | 31.94 | 14.09 |
| 17 | 22.23 | 16.18 | / | / | / |

In some embodiments of the present application, the crystalline form I of the hydrochloride of the compound of formula (I) has an X-ray powder diffraction (XRPD) pattern using Cu Kα radiation as shown in FIG. 4.

In some embodiments of the present application, the crystalline form I of the hydrochloride of the compound of formula (I) has a differential scanning calorimetry (DSC) curve showing an endothermic peak at 204.5±3 °C.

In some embodiments of the present application, the crystalline form I of the hydrochloride of the compound of formula (I) has a DSC pattern as shown in FIG. 5.

In some embodiments of the present application, the crystalline form I of the hydrochloride of the compound of formula (I) has a thermogravimetric analysis (TGA) curve showing a weight loss of 2.28% at 150.0±3 °C.

In some embodiments of the present application, the crystalline form I of the hydrochloride of the compound of formula (I) has a TGA pattern as shown in FIG. 5.

In another aspect, the present application provides a method for preparing the crystalline form I of the hydrochloride of the compound of formula (I), comprising a step of precipitating the crystalline form of the hydrochloride of the compound of formula (I) in a mixed solvent of ethyl acetate and water.

In some embodiments of the present application, the crystalline form I of the hydrochloride of the compound of formula (I) of the present application is prepared by a method comprising:

(1) reacting the compound of formula (I) with hydrochloric acid in the presence of ethyl acetate and water to give the hydrochloride of the compound of formula (I); and

(2) crystallization.

In some embodiments of the present application, in the crystalline form I of the hydrochloride of the compound of formula (I), a molar ratio of the compound of formula (I) to hydrochloric acid is 1:1, or the hydrochloride of the compound of formula (I) is the compound of formula (II):

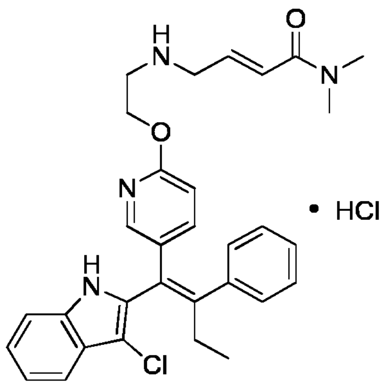

(II)

In some embodiments of the present application, provided is a method for preparing the crystalline form I of the compound of formula (II), comprising: mixing the crystalline form of the compound of formula (I) with ethyl acetate, heating until the solution becomes clear, adding a solution of hydrogen chloride in ethyl acetate and stirring, cooling for crystallization, and then filtering and drying under reduced pressure to give the crystalline form I of the compound of formula (II).

In another aspect, the present application further provides a crystalline form II of the hydrochloride of the compound of formula (I).

In some embodiments of the present application, the crystalline form II of the hydrochloride of the compound of formula (I) has diffraction peaks in an X-ray powder diffraction pattern using Cu Kα radiation at 2Θ values of 9.84±0.20°, 16.76±0.20°, and 20.09±0.20°.

In some embodiments of the present application, the crystalline form II of the hydrochloride of the compound of formula (I) has diffraction peaks in an X-ray powder diffraction pattern using Cu Kα radiation at 2Θ values of 9.84±0.20°, 16.31±0.20°, 16.76±0.20°, 20.09±0.20°, 22.61±0.20°, 23.65±0.20°, 24.55±0.20°, and 25.32±0.20°.

In some embodiments of the present application, the crystalline form II of the hydrochloride of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 9.84±0.20°, 10.59±0.20°, 12.39±0.20°, 12.61±0.20°, 13.99±0.20°, 16.31±0.20°, 16.76±0.20°, 18.31±0.20°, 18.72±0.20°, 19.06±0.20°, 19.55±0.20°, 19.72±0.20°, 20.09±0.20°, 20.77±0.20°, 21.22±0.20°, 22.61±0.20°, 23.04±0.20°, 23.41±0.20°, 23.65±0.20°, 24.55±0.20°, 25.32±0.20°, 26.49±0.20°, 26.90±0.20°, 27.71±0.20°, 28.27±0.20°, 28.47±0.20°, 29.22±0.20°, 29.61±0.20°, 30.22±0.20°, 31.11±0.20°, 31.47±0.20°, and 34.18±0.20°.

In some embodiments of the present application, the crystalline form II of the hydrochloride of the compound of formula (I) comprises 3, 4, 5, 6, 7, or 8 diffraction peaks in an X-ray powder diffraction pattern using Cu Kα radiation at 2Θ values selected from the group consisting of: 9.84±0.20°, 16.31±0.20°, 16.76±0.20°, 20.09±0.20°, 22.61±0.20°, 23.65±0.20°, 24.55±0.20°, and 25.32±0.20°.

In some embodiments of the present application, the crystalline form II of the hydrochloride of the compound of formula (I) has diffraction peaks in an X-ray powder diffraction pattern using Cu Kα radiation at 2Θ values of about 9.84°, 10.59°, 12.39°, 12.61°, 13.99°, 16.31°, 16.76°, 18.31°, 18.72°, 19.06°, 19.55°, 19.72°, 20.09°, 20.77°, 21.22°, 22.61°, 23.04°, 23.41°, 23.65°, 24.55°, 25.11°, 25.32°, 26.49°, 26.90°, 27.71°, 28.27°, 28.47°, 29.22°, 29.61°, 30.22°, 31.11°, 31.47°, and 34.18°.

In some embodiments of the present application, in an X-ray powder diffraction pattern of the crystalline form II of the hydrochloride of the compound of formula (I) using Cu Kα radiation, the peak positions and relative intensities of diffraction peaks are shown in Table 10 below:

**Table 10. Peak positions and relative intensities of diffraction peaks in the X-ray powder diffraction pattern of the crystalline form II of the hydrochloride of the compound of formula (I)**

| No. | 2θ[°]±0.20° | Relative intensity [%] | No. | 2θ[°]±0.20° | Relative intensity [%] |
|---|---|---|---|---|---|
| 1 | 9.84 | 64.71 | 18 | 23.41 | 33.33 |
| 2 | 10.59 | 12.48 | 19 | 23.65 | 53.09 |
| 3 | 12.39 | 19.68 | 20 | 24.55 | 30.34 |
| 4 | 12.61 | 13.03 | 21 | 25.11 | 22.26 |
| 5 | 13.99 | 9.69 | 22 | 25.32 | 48.21 |
| 6 | 16.31 | 36.47 | 23 | 26.49 | 7.41 |
| 7 | 16.76 | 100.00 | 24 | 26.90 | 9.23 |
| 8 | 18.31 | 12.27 | 25 | 27.71 | 12.85 |
| 9 | 18.72 | 16.61 | 26 | 28.27 | 15.58 |
| 10 | 19.06 | 21.17 | 27 | 28.47 | 20.52 |
| 11 | 19.55 | 21.47 | 28 | 29.22 | 7.04 |
| 12 | 19.72 | 31.12 | 29 | 29.61 | 9.84 |
| 13 | 20.09 | 64.32 | 30 | 30.22 | 8.19 |
| 14 | 20.77 | 16.93 | 31 | 31.11 | 8.95 |
| 15 | 21.22 | 6.77 | 32 | 31.47 | 3.76 |
| 16 | 22.61 | 45.91 | 33 | 34.18 | 3.40 |
| 17 | 23.04 | 27.09 | / | / | / |

In some embodiments of the present application, the crystalline form II of the hydrochloride of the compound of formula (I) has an X-ray powder diffraction (XRPD) pattern using Cu Kα radiation as shown in FIG. 17.

In some embodiments of the present application, the crystalline form II of the hydrochloride of the compound of formula (I) has a differential scanning calorimetry (DSC) curve showing endothermic peaks at 101.4±3°C, 113.3±3°C, and 195.9±3°C.

In some embodiments of the present application, the crystalline form II of the hydrochloride of the compound of formula (I) has a DSC pattern as shown in FIG. 18.

In some embodiments of the present application, the crystalline form II of the hydrochloride of the compound of formula (I) has a thermogravimetric analysis (TGA) curve showing a weight loss of 5.48% at 55.0±3 °C and 9.67% at 90.0±3°C.

In some embodiments of the present application, the crystalline form II of the hydrochloride of the compound of formula (I) has a TGA pattern as shown in FIG. 18.

In another aspect, the present application provides a method for preparing the crystalline form of the hydrochloride of the compound of formula (I), comprising a step of precipitating a crystalline form II of the compound of formula

(II) in a mixed solvent of ethyl acetate and water.

In some embodiments of the present application, the crystalline form II of the hydrochloride of the compound of formula (I) of the present application is prepared by a method comprising:

(1) reacting the crystalline form I of the hydrochloride of the compound of formula (I) in the presence of ethyl acetate and water; and
(2) crystallization.

In some embodiments of the present application, in the crystalline form II of the hydrochloride of the compound of formula (I), a molar ratio of the compound of formula (I) to hydrochloric acid is 1:1, or the hydrochloride of the compound of formula (I) is the compound of formula (II):

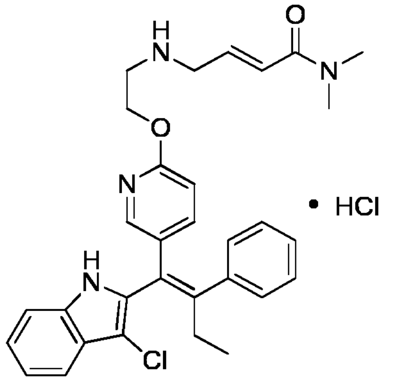

(II)

.

In some embodiments of the present application, provided is a method for preparing the crystalline form II of the compound of formula (II), comprising: mixing the crystalline form I of the compound of formula (II) with ethyl acetate and water, heating and stirring until the solution becomes clear, cooling for crystallization, and then filtering and drying under reduced pressure to give the crystalline form II of the compound of formula (II).

In another aspect, the present application provides a fumarate of the compound of formula (I).

In some embodiments of the present application, the fumarate of the compound of formula (I) is in a crystalline form.

In another aspect, the present application provides a crystalline form of the fumarate of the compound of formula (I).

In some embodiments of the present application, the crystalline form of the fumarate of the compound of formula (I) has diffraction peaks in an X-ray powder diffraction pattern using Cu Kα radiation at 2Θ values of 5.12±0.20°, 14.99±0.20°, and 19.17±0.20°.

In some embodiments of the present application, the crystalline form of the fumarate of the compound of formula (I) has diffraction peaks in an X-ray powder diffraction pattern using Cu Kα radiation at 2Θ values of 5.12±0.20°, 9.20±0.20°, 14.99±0.20°, 18.08±0.20°, 19.17±0.20°, 21.39±0.20°, 22.57±0.20°, and 25.15±0.20°.

In some embodiments of the present application, the crystalline form of the fumarate of the compound of formula (I) comprises 3, 4, 5, 6, 7, 8 or more diffraction peaks in an X-ray powder diffraction pattern using Cu Kα radiation at 2Θ values selected from the group consisting of: 5.12±0.20°, 9.20±0.20°, 12.03±0.20°, 14.99±0.20°, 15.31±0.20°, 16.75±0.20°, 17.62±0.20°, 18.08±0.20°, 18.83±0.20°, 19.17±0.20°, 20.80±0.20°, 21.39±0.20°, 22.21±0.20°, 22.57±0.20°, 23.09±0.20°, 23.50±0.20°, 24.42±0.20°, 25.15±0.20°, 25.78±0.20°, 27.14±0.20°, 28.25±0.20°, 29.54±0.20°, 30.50±0.20°, 31.09±0.20°, and 32.16±0.20°.

In some embodiments of the present application, the crystalline form of the fumarate of the compound of formula (I) comprises 3, 4, 5, 6, 7, or 8 diffraction peaks in an X-ray powder diffraction pattern using Cu Kα radiation at 2Θ values selected from the group consisting of: 5.12±0.20°, 9.20±0.20°, 14.99±0.20°, 18.08±0.20°, 19.17±0.20°, 21.39±0.20°, 22.57±0.20°, and 25.15±0.20°.

In some embodiments of the present application, the crystalline form of the fumarate of the compound of formula (I) has diffraction peaks in an X-ray powder diffraction pattern using Cu Kα radiation at 2Θ values of about 5.12°, 9.20°, 12.03°, 14.99°, 15.31°, 16.75°, 17.62°, 18.08°, 18.83°, 19.17°, 20.80°, 21.39°, 22.21°, 22.57°, 23.09°, 23.50°, 24.42°, 25.15°, 25.78°, 27.14°, 28.25°, 29.54°, 30.50°, 31.09°, and 32.16°.

In some embodiments of the present application, in an X-ray powder diffraction pattern of the crystalline form of the fumarate of the compound of formula (I) using Cu Kα radiation, the peak positions and relative intensities of

diffraction peaks are shown in Table 3 below:

**Table 3. Peak positions and relative intensities of diffraction peaks in the X-ray powder diffraction pattern of the crystalline form of the fumarate of the compound of formula (I)**

| No. | 2θ[°]±0.20° | Relative intensity [%] | No. | 2θ[°]±0.20° | Relative intensity [%] |
|---|---|---|---|---|---|
| 1 | 5.12 | 51.21 | 14 | 22.57 | 31.65 |
| 2 | 9.20 | 32.34 | 15 | 23.09 | 16.96 |
| 3 | 12.03 | 18.66 | 16 | 23.50 | 15.38 |
| 4 | 14.99 | 100.00 | 17 | 24.42 | 9.36 |
| 5 | 15.31 | 84.38 | 18 | 25.15 | 22.41 |
| 6 | 16.75 | 13.91 | 19 | 25.78 | 11.69 |
| 7 | 17.62 | 10.08 | 20 | 27.14 | 13.27 |
| 8 | 18.08 | 22.14 | 21 | 28.25 | 4.89 |
| 9 | 18.83 | 60.85 | 22 | 29.54 | 3.01 |
| 10 | 19.17 | 96.79 | 23 | 30.50 | 6.10 |
| 11 | 20.80 | 17.86 | 24 | 31.09 | 6.76 |
| 12 | 21.39 | 30.39 | 25 | 32.16 | 5.58 |
| 13 | 22.21 | 18.37 | / | / | / |

In some embodiments of the present application, the crystalline form of the fumarate of the compound of formula (I) has an X-ray powder diffraction (XRPD) pattern using Cu Kα radiation as shown in FIG. 7.

In some embodiments of the present application, the crystalline form of the fumarate of the compound of formula (I) has a differential scanning calorimetry (DSC) curve using Cu Kα radiation showing an endothermic peak at 164.5±3 °C.

In some embodiments of the present application, the crystalline form of the fumarate of the compound of formula (I) has a DSC pattern as shown in FIG. 8.

In some embodiments of the present application, the crystalline form of the fumarate of the compound of formula (I) has a thermogravimetric analysis (TGA) curve showing a weight loss of 0.85% at 140.0±3 °C.

In some embodiments of the present application, the crystalline form of the fumarate of the compound of formula (I) has a TGA pattern as shown in FIG. 8.

In another aspect, the present application provides a method for preparing the crystalline form of the fumarate of the compound of formula (I), comprising a step of precipitating the crystalline form of the fumarate of the compound of formula (I) in a solvent of acetone.

In some embodiments of the present application, the crystalline form of the fumarate of the compound of formula (I) of the present application is prepared by a method comprising:

(1) reacting the compound of formula (I) with fumaric acid in the presence of acetone to give the fumarate of the compound of formula (I); and
(2) crystallization.

In some embodiments of the present application, in the fumarate of the compound of formula (I) or the crystalline form of the fumarate of the compound of formula (I), a molar ratio of the compound of formula (I) to fumaric acid is 1:1, or the fumarate of the compound of formula (I) is the compound of formula (III):

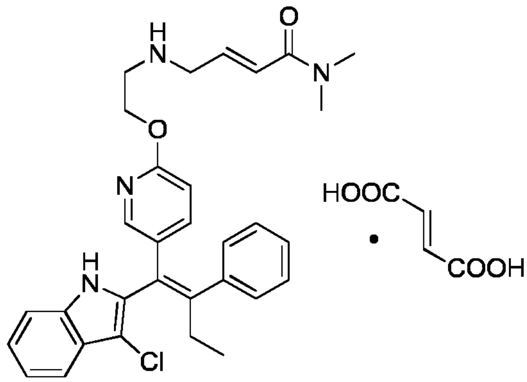

(III)

In some embodiments of the present application, provided is a method for preparing the crystalline form of the compound of formula (III), comprising: mixing the crystalline form of the compound of formula (I) with acetone, heating until the solution becomes clear, adding fumaric acid and stirring, cooling for crystallization, and then filtering and drying under reduced pressure to give the crystalline form of the compound of formula (III).

In another aspect, the present application further provides a maleate of the compound of formula (I).

In some embodiments of the present application, the maleate of the compound of formula (I) is in a crystalline form.

In another aspect, the present application further provides the crystalline form of the maleate of the compound of formula (I).

In some embodiments of the present application, the crystalline form of the maleate of the compound of formula (I) has diffraction peaks in an X-ray powder diffraction pattern using Cu Kα radiation at 2Θ values of 4.55±0.20°, 18.12±0.20°, and 21.18±0.20°.

In some embodiments of the present application, the crystalline form of the maleate of the compound of formula (I) has diffraction peaks in an X-ray powder diffraction pattern using Cu Kα radiation at 2Θ values of 4.55±0.20°, 12.85±0.20°, 16.19±0.20°, 16.68±0.20°, 18.12±0.20°, 21.18±0.20°, 22.71±0.20°, and 27.31±0.20°.

In some embodiments of the present application, the crystalline form of the maleate of the compound of formula (I) comprises 3, 4, 5, 6, 7, 8 or more diffraction peaks in an X-ray powder diffraction pattern using Cu Kα radiation at 2Θ values selected from the group consisting of: 4.55±0.20°, 9.06±0.20°, 10.63±0.20°, 11.00±0.20°, 12.85±0.20°, 13.75±0.20°, 15.96±0.20°, 16.19±0.20°, 16.68±0.20°, 16.99±0.20°, 17.51±0.20°, 18.12±0.20°, 20.12±0.20°, 20.75±0.20°, 21.18±0.20°, 22.71±0.20°, 23.04±0.20°, 24.13±0.20°, 24.55±0.20°, 25.32±0.20°, 25.95±0.20°, 27.31±0.20°, 28.38±0.20°, 28.97±0.20°, 29.62±0.20°, and 34.09±0.20°.

In some embodiments of the present application, the crystalline form of the maleate of the compound of formula (I) comprises 3, 4, 5, 6, 7, or 8 diffraction peaks in an X-ray powder diffraction pattern using Cu Kα radiation at 2Θ values selected from the group consisting of: 4.55±0.20°, 12.85±0.20°, 16.19±0.20°, 16.68±0.20°, 18.12±0.20°, 21.18±0.20°, 22.71±0.20°, and 27.31±0.20°.

In some embodiments of the present application, the crystalline form of the maleate of the compound of formula (I) has diffraction peaks in an X-ray powder diffraction pattern using Cu Kα radiation at 2Θ values of about 4.55°, 9.06°, 10.63°, 11.00°, 12.85°, 13.75°, 15.96°, 16.19°, 16.68°, 16.99°, 17.51°, 18.12°, 20.12°, 20.75°, 21.18°, 22.71°, 23.04°, 24.13°, 24.55°, 25.32°, 25.95°, 27.31°, 28.38°, 28.97°, 29.62°, and 34.09°.

In some embodiments of the present application, in an X-ray powder diffraction pattern of the crystalline form of the maleate of the compound of formula (I) using Cu Kα radiation, the peak positions and relative intensities of diffraction peaks are shown in Table 4 below:

**Table 4. Peak positions and relative intensities of diffraction peaks in the X-ray powder diffraction pattern of the crystalline form of the maleate of the compound of formula (I)**

| No. | 2θ[°]±0.20° | Relative intensity [%] | No. | 2θ[°]±0.20° | Relative intensity [%] |
|---|---|---|---|---|---|
| 1 | 4.55 | 100.00 | 14 | 20.75 | 15.22 |
| 2 | 9.06 | 11.80 | 15 | 21.18 | 47.91 |
| 3 | 10.63 | 10.55 | 16 | 22.71 | 29.26 |

(continued)

| No. | 2θ[°]±0.20° | Relative intensity [%] | No. | 2θ[°]±0.20° | Relative intensity [%] |
|---|---|---|---|---|---|
| 4 | 11.00 | 2.59 | 17 | 23.04 | 10.23 |
| 5 | 12.85 | 22.23 | 18 | 24.13 | 6.01 |
| 6 | 13.75 | 10.12 | 19 | 24.55 | 12.35 |
| 7 | 15.96 | 14.81 | 20 | 25.32 | 11.64 |
| 8 | 16.19 | 25.24 | 21 | 25.95 | 12.50 |
| 9 | 16.68 | 28.47 | 22 | 27.31 | 33.85 |
| 10 | 16.99 | 13.63 | 23 | 28.38 | 6.33 |
| 11 | 17.51 | 21.12 | 24 | 28.97 | 10.68 |
| 12 | 18.12 | 59.74 | 25 | 29.62 | 4.42 |
| 13 | 20.12 | 7.02 | 26 | 34.09 | 6.54 |

In some embodiments of the present application, the crystalline form of the maleate of the compound of formula (I) has an X-ray powder diffraction (XRPD) pattern using Cu Kα radiation as shown in FIG. 9.

In some embodiments of the present application, the crystalline form of the maleate of the compound of formula (I) has a differential scanning calorimetry (DSC) curve showing an endothermic peak at 160.3±3 °C.

In some embodiments of the present application, the crystalline form of the maleate of the compound of formula (I) has a DSC pattern as shown in FIG. 10.

In some embodiments of the present application, the crystalline form of the maleate of the compound of formula (I) has a thermogravimetric analysis (TGA) curve showing a weight loss of 1.67% at 140.0±3 °C.

In some embodiments of the present application, the crystalline form of the maleate of the compound of formula (I) has a TGA pattern as shown in FIG. 10.

In another aspect, the present application provides a method for preparing the crystalline form of the maleate of the compound of formula (I), comprising a step of precipitating the crystalline form of the maleate of the compound of formula (I) in a solvent of ethyl acetate.

In some embodiments of the present application, the crystalline form of the maleate of the compound of formula (I) of the present application is prepared by a method comprising:

(1) reacting the compound of formula (I) with maleic acid in the presence of ethyl acetate to give the maleate of the compound of formula (I); and
(2) crystallization.

In some embodiments of the present application, in the maleate of the compound of formula (I) or the crystalline form of the maleate of the compound of formula (I), a molar ratio of the compound of formula (I) to maleic acid is 1:1, or the maleate of the compound of formula (I) is the compound of formula (IV):

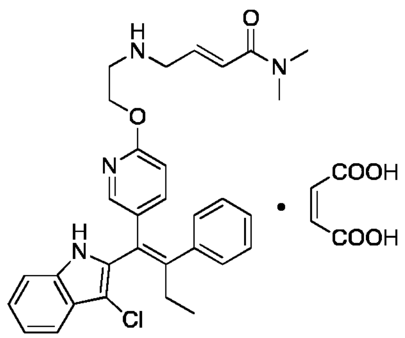

(IV)

In some embodiments of the present application, provided is a method for preparing the crystalline form of the

compound of formula (IV), comprising: mixing the crystalline form of the compound of formula (I) with ethyl acetate and heating until the solution becomes clear, adding maleic acid and stirring, cooling for crystallization, and then filtering and drying under reduced pressure to give the crystalline form of the compound of formula (IV). In another aspect, the present application further provides a methanesulfonate of the compound of formula (I).

In some embodiments of the present application, the methanesulfonate of the compound of formula (I) is in a crystalline form.

In another aspect, the present application further provides a crystalline form I of the methanesulfonate of the compound of formula (I).

In some embodiments of the present application, the crystalline form I of the methanesulfonate of the compound of formula (I) has diffraction peaks in an X-ray powder diffraction pattern using Cu K$\alpha$ radiation at 2$\Theta$ values of 5.47±0.20°, 6.25±0.20°, and 16.11±0.20°.

In some embodiments of the present application, the crystalline form I of the methanesulfonate of the compound of formula (I) has diffraction peaks in an X-ray powder diffraction pattern using Cu K$\alpha$ radiation at 2$\Theta$ values of 5.47±0.20°, 6.25±0.20°, 16.11±0.20°, 16.64±0.20°, 18.11±0.20°, 19.73±0.20°, 24.22±0.20°, and 25.12±0.20°.

In some embodiments of the present application, the crystalline form I of the methanesulfonate of the compound of formula (I) comprises 3, 4, 5, 6, 7, 8 or more diffraction peaks in an X-ray powder diffraction pattern using Cu K$\alpha$ radiation at 2$\Theta$ values selected from the group consisting of: 5.47±0.20°, 6.00±0.20°, 6.25±0.20°, 8.29±0.20°, 9.62±0.20°, 10.88±0.20°, 12.02±0.20°, 12.46±0.20°, 13.81±0.20°, 16.11±0.20°, 16.39±0.20°, 16.64±0.20°, 18.11±0.20°, 18.72±0.20°, 19.73±0.20°, 20.21±0.20°, 21.08±0.20°, 21.41±0.20°, 22.80±0.20°, 23.45±0.20°, 24.22±0.20°, 24.55±0.20°, 25.12±0.20°, 25.64±0.20°, 26.00±0.20°, 26.99±0.20°, 29.06±0.20°, 29.88±0.20°, and 31.18±0.20°.

In some embodiments of the present application, the crystalline form I of the methanesulfonate of the compound of formula (I) comprises 3, 4, 5, 6, 7, or 8 diffraction peaks in an X-ray powder diffraction pattern using Cu K$\alpha$ radiation at 2$\Theta$ values selected from the group consisting of: 5.47±0.20°, 6.25±0.20°, 16.11±0.20°, 16.64±0.20°, 18.11±0.20°, 19.73±0.20°, 24.22±0.20°, and 25.12±0.20°.

In some embodiments of the present application, the crystalline form I of the methanesulfonate of the compound of formula (I) has diffraction peaks in an X-ray powder diffraction pattern using Cu K$\alpha$ radiation at 2$\Theta$ values of about 5.47°, 6.00°, 6.25°, 8.29°, 9.62°, 10.88°, 12.02°, 12.46°, 13.81°, 16.11°, 16.39°, 16.64°, 18.11°, 18.72°, 19.73°, 20.21°, 21.08°, 21.41°, 22.80°, 23.45°, 24.22°, 24.55°, 25.12°, 25.64°, 26.00°, 26.99°, 29.06°, 29.88°, and 31.18°.

In some embodiments of the present application, in an X-ray powder diffraction pattern of the crystalline form I of the methanesulfonate of the compound of formula (I) using Cu K$\alpha$ radiation, the peak positions and relative intensities of diffraction peaks are shown in Table 5 below:

**Table 5. Peak positions and relative intensities of diffraction peaks of the crystalline form I of the methanesulfonate of the compound of formula (I)**

| No. | 2θ[°]±0.20° | Relative intensity [%] | No. | 2θ[°]±0.20° | Relative intensity [%] |
|---|---|---|---|---|---|
| 1 | 5.47 | 74.01 | 16 | 20.21 | 24.03 |
| 2 | 6.00 | 60.97 | 17 | 21.08 | 28.68 |
| 3 | 6.25 | 100.00 | 18 | 21.41 | 26.96 |
| 4 | 8.29 | 10.69 | 19 | 22.80 | 23.66 |
| 5 | 9.62 | 18.63 | 20 | 23.45 | 18.30 |
| 6 | 10.88 | 15.01 | 21 | 24.22 | 30.77 |
| 7 | 12.02 | 12.10 | 22 | 24.55 | 25.63 |
| 8 | 12.46 | 6.06 | 23 | 25.12 | 39.39 |
| 9 | 13.81 | 14.98 | 24 | 25.64 | 19.77 |
| 10 | 16.11 | 48.43 | 25 | 26.00 | 13.35 |
| 11 | 16.39 | 29.24 | 26 | 26.99 | 8.58 |
| 12 | 16.64 | 31.25 | 27 | 29.06 | 13.79 |
| 13 | 18.11 | 39.27 | 28 | 29.88 | 4.07 |
| 14 | 18.72 | 20.06 | 29 | 31.18 | 6.58 |

(continued)

| No. | 2θ[°]±0.20° | Relative intensity [%] | No. | 2θ[°]±0.20° | Relative intensity [%] |
|---|---|---|---|---|---|
| 15 | 19.73 | 35.59 | / | / | / |

In some embodiments of the present application, the crystalline form I of the methanesulfonate of the compound of formula (I) has an X-ray powder diffraction (XRPD) pattern using Cu Kα radiation as shown in FIG. 11.

In another aspect, the present application provides a method for preparing the crystalline form I of the methanesulfonate of the compound of formula (I), comprising a step of precipitating the crystalline form I of the methanesulfonate of the compound of formula (I) in a solvent of ethyl acetate.

In some embodiments of the present application, the crystalline form I of the methanesulfonate of the compound of formula (I) of the present application is prepared by a method comprising:

(1) reacting the compound of formula (I) with methanesulfonic acid in the presence of ethyl acetate to give the methanesulfonate of the compound of formula (I); and
(2) crystallization.

In another aspect, the present application further provides a crystalline form II of the methanesulfonate of the compound of formula (I).

In some embodiments of the present application, the crystalline form II of the methanesulfonate of the compound of formula (I) has diffraction peaks in an X-ray powder diffraction pattern using Cu Kα radiation at 2Θ values of 6.17±0.20°, 8.76±0.20°, and 23.03±0.20°.

In some embodiments of the present application, the crystalline form II of the methanesulfonate of the compound of formula (I) has diffraction peaks in an X-ray powder diffraction pattern using Cu Kα radiation at 2Θ values of 6.17±0.20°, 8.76±0.20°, 12.16±0.20°, 16.12±0.20°, 17.18±0.20°, 19.23±0.20°, 20.19±0.20°, and 23.03±0.20°.

In some embodiments of the present application, the crystalline form II of the methanesulfonate of the compound of formula (I) comprises 3, 4, 5, 6, 7, 8 or more diffraction peaks in an X-ray powder diffraction pattern using Cu Kα radiation at 2Θ values selected from the group consisting of: 6.17±0.20°, 8.76±0.20°, 12.16±0.20°, 12.37±0.20°, 14.53±0.20°, 15.46±0.20°, 16.12±0.20°, 17.18±0.20°, 17.40±0.20°, 18.30±0.20°, 18.78±0.20°, 19.23±0.20°, 19.71±0.20°, 20.19±0.20°, 20.74±0.20°, 21.05±0.20°, 22.19±0.20°, and 23.03±0.20°.

In some embodiments of the present application, the crystalline form II of the methanesulfonate of the compound of formula (I) comprises 3, 4, 5, 6, 7, or 8 diffraction peaks in an X-ray powder diffraction pattern using Cu Kα radiation at 2Θ values selected from the group consisting of: 6.17±0.20°, 8.76±0.20°, 12.16±0.20°, 16.12±0.20°, 17.18±0.20°, 19.23±0.20°, 20.19±0.20°, and 23.03±0.20°.

In some embodiments of the present application, the crystalline form II of the methanesulfonate of the compound of formula (I) has diffraction peaks in an X-ray powder diffraction pattern using Cu Kα radiation at 2Θ values of about 6.17°, 8.76°, 12.16°, 12.37°, 14.53°, 15.46°, 16.12°, 17.18°, 17.40°, 18.30°, 18.78°, 19.23°, 19.71°, 20.19°, 20.74°, 21.05°, 22.19°, and 23.03°.

In some embodiments of the present application, in an X-ray powder diffraction pattern of the crystalline form II of the methanesulfonate of the compound of formula (I) using Cu Kα radiation, the peak positions and relative intensities of diffraction peaks are shown in Table 6 below:

**Table 6. Peak positions and relative intensities of diffraction peaks of the crystalline form II of the methanesulfonate of the compound of formula (I)**

| No. | 2θ[°]±0.20° | Relative intensity [%] | No. | 2θ[°]±0.20° | Relative intensity [%] |
|---|---|---|---|---|---|
| 1 | 6.17 | 100 | 10 | 18.3 | 4.78 |
| 2 | 8.76 | 20.11 | 11 | 18.78 | 2.35 |
| 3 | 12.16 | 10.48 | 12 | 19.23 | 5.56 |
| 4 | 12.37 | 7.44 | 13 | 19.71 | 4.55 |
| 5 | 14.53 | 2.52 | 14 | 20.19 | 5.94 |
| 6 | 15.46 | 2.38 | 15 | 20.74 | 5.51 |

(continued)

| No. | 2θ[°]±0.20° | Relative intensity [%] | No. | 2θ[°]±0.20° | Relative intensity [%] |
|---|---|---|---|---|---|
| 7 | 16.12 | 14.16 | 16 | 21.05 | 5.29 |
| 8 | 17.18 | 14.45 | 17 | 22.19 | 2.67 |
| 9 | 17.4 | 11.49 | 18 | 23.03 | 14.77 |

In some embodiments of the present application, the crystalline form II of the methanesulfonate of the compound of formula (I) has an X-ray powder diffraction (XRPD) pattern using Cu Kα radiation as shown in FIG. 12.

In another aspect, the present application provides a method for preparing the crystalline form II of the methanesulfonate of the compound of formula (I), comprising a step of precipitating the crystalline form II of the methanesulfonate of the compound of formula (I) in a solvent of acetone.

In some embodiments of the present application, the crystalline form II of the methanesulfonate of the compound of formula (I) of the present application is prepared by a method comprising:

(1) reacting the compound of formula (I) with methanesulfonic acid in the presence of acetone to give the methanesulfonate of the compound of formula (I); and
(2) crystallization.

In some embodiments of the present application, in the methanesulfonate of the compound of formula (I), the crystalline form I of the methanesulfonate of the compound of formula (I), or the crystalline form II of the methanesulfonate of the compound of formula (I), a molar ratio of the compound of formula (I) to methanesulfonic acid is 1:2, or the methanesulfonate of the compound of formula (I) is the compound of formula (V):

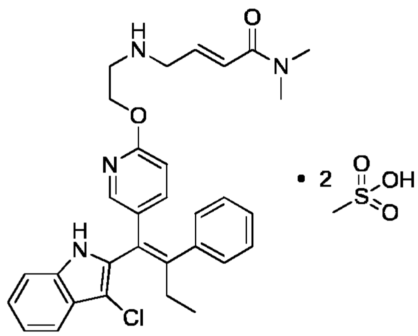

(V)

In some embodiments of the present application, provided is a method for preparing the crystalline form I of the compound of formula (V), comprising: mixing the crystalline form of the compound of formula (I) with ethyl acetate and heating until the solution becomes clear, adding methanesulfonic acid and stirring, cooling for crystallization, and then filtering and drying under reduced pressure to give the crystalline form I of the compound of formula (V).

In some embodiments of the present application, provided is a method for preparing the crystalline form II of the compound of formula (V), comprising: mixing the crystalline form of the compound of formula (I) with acetone and heating until the solution becomes clear, adding methanesulfonic acid and stirring, cooling for crystallization, and then filtering and drying under reduced pressure to give the crystalline form II of the compound of formula (V).

In another aspect, the present application further provides a hydrobromide of the compound of formula (I).

In some embodiments of the present application, the hydrobromide of the compound of formula (I) is in a crystalline form.

In another aspect, the present application further provides the crystalline form of the hydrobromide of the compound of formula (I).

In some embodiments of the present application, the crystalline form of the hydrobromide of the compound of formula (I) has diffraction peaks in an X-ray powder diffraction pattern using Cu Kα radiation at 2Θ values of 7.59±0.20°, 17.62±0.20°, and 23.84±0.20°.

In some embodiments of the present application, the crystalline form of the hydrobromide of the compound of formula (I) has diffraction peaks in an X-ray powder diffraction pattern using Cu Kα radiation at 2Θ values of 3.12±0.20°,

5.96±0.20°, 7.59±0.20°, 16.49±0.20°, 17.62±0.20°, 23.84±0.20°, 24.54±0.20°, and 30.30±0.20°.

In some embodiments of the present application, the crystalline form of the hydrobromide of the compound of formula (I) comprises 3, 4, 5, 6, 7, 8 or more diffraction peaks in an X-ray powder diffraction pattern using Cu Kα radiation at 2Θ values selected from the group consisting of: 3.12±0.20°, 5.96±0.20°, 7.59±0.20°, 9.76±0.20°, 11.11±0.20°, 12.78±0.20°, 16.49±0.20°, 17.62±0.20°, 18.44±0.20°, 19.57±0.20°, 20.69±0.20°, 21.16±0.20°, 22.16±0.20°, 22.77±0.20°, 23.84±0.20°, 24.31±0.20°, 24.54±0.20°, 25.15±0.20°, 26.07±0.20°, 27.31±0.20°, 27.83±0.20°, 28.91±0.20°, 30.30±0.20°, and 31.74±0.20°.

In some embodiments of the present application, the crystalline form of the hydrobromide of the compound of formula (I) comprises 3, 4, 5, 6, 7, or 8 diffraction peaks in an X-ray powder diffraction pattern using Cu Kα radiation at 2Θ values selected from the group consisting of: 3.12±0.20°, 5.96±0.20°, 7.59±0.20°, 16.49±0.20°, 17.62±0.20°, 23.84±0.20°, 24.54±0.20°, and 30.30±0.20°.

In some embodiments of the present application, the crystalline form of the hydrobromide of the compound of formula (I) has diffraction peaks in an X-ray powder diffraction pattern using Cu Kα radiation at 2Θ values of about 3.12°, 5.96°, 7.59°, 9.76°, 11.11°, 12.78°, 16.49°, 17.62°, 18.44°, 19.57°, 20.69°, 21.16°, 22.16°, 22.77°, 23.84°, 24.31°, 24.54°, 25.15°, 26.07°, 27.31°, 27.83°, 28.91°, 30.30°, and 31.74°.

In some embodiments of the present application, in an X-ray powder diffraction pattern of the crystalline form of the hydrobromide of the compound of formula (I) using Cu Kα radiation, the peak positions and relative intensities of diffraction peaks are shown in Table 7 below:

**Table 7. Peak positions and relative intensities of diffraction peaks of the crystalline form of the hydrobromide of the compound of formula (I)**

| No. | 2θ[°]±0.20° | Relative intensity [%] | No. | 2θ[°]±0.20° | Relative intensity [%] |
|---|---|---|---|---|---|
| 1 | 5.96 | 20.18 | 13 | 22.77 | 9.6 |
| 2 | 7.59 | 100 | 14 | 23.84 | 55.62 |
| 3 | 9.76 | 8.08 | 15 | 24.31 | 17.23 |
| 4 | 11.11 | 12.41 | 16 | 24.54 | 28.87 |
| 5 | 12.78 | 8.2 | 17 | 25.15 | 5.35 |
| 6 | 16.49 | 29.84 | 18 | 26.07 | 6.27 |
| 7 | 17.62 | 36.58 | 19 | 27.31 | 8.24 |
| 8 | 18.44 | 9.26 | 20 | 27.83 | 5.72 |
| 9 | 19.57 | 8.24 | 21 | 28.91 | 7.79 |
| 10 | 20.69 | 7.49 | 22 | 30.3 | 14.96 |
| 11 | 21.16 | 11.38 | 23 | 31.74 | 11.81 |
| 12 | 22.16 | 4.85 | / | / | / |

In some embodiments of the present application, the crystalline form of the hydrobromide of the compound of formula (I) has an X-ray powder diffraction (XRPD) pattern using Cu Kα radiation as shown in FIG. 13.

In another aspect, the present application provides a method for preparing the crystalline form of the hydrobromide of the compound of formula (I), comprising a step of precipitating the crystalline form of the hydrobromide of the compound of formula (I) in a mixed solvent of acetone and water.

In some embodiments of the present application, the crystalline form of the hydrobromide of the compound of formula (I) of the present application is prepared by a method comprising:

(1) reacting the compound of formula (I) with hydrobromic acid in the presence of acetone and water to give the hydrobromide of the compound of formula (I); and
(2) crystallization.

In some embodiments of the present application, in the hydrobromide of the compound of formula (I) or the crystalline form of the hydrobromide of the compound of formula (I), a molar ratio of the compound of formula (I) to hydrobromic acid is 1:1, or the hydrobromide of the compound of formula (I) is the compound of formula (VI):

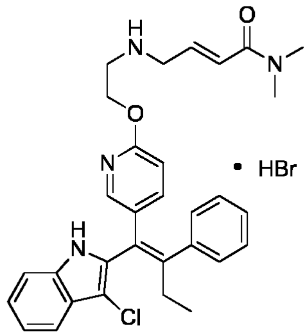

(VI)

In some embodiments of the present application, provided is a method for preparing the crystalline form I of the compound of formula (VI), comprising: mixing the crystalline form of the compound of formula (I) with acetone and heating until the solution becomes clear, adding hydrobromic acid and stirring, cooling for crystallization, and then filtering and drying under reduced pressure to give the crystalline form I of the compound of formula (VI).

In another aspect, the present application provides a method for preparing a crystalline form of a salt of the compound of formula (I), comprising:

(1) mixing the crystalline form of the compound of formula (I) with a solvent selected from the group consisting of acetone and ethyl acetate and heating until the solution becomes clear; and

(2) adding an acid for salt formation to the solution described above and stirring, cooling for crystallization, and then filtering and drying under reduced pressure to give the crystalline form of the salt of the compound of formula (I).

Alternatively, the present application provides an additional method for preparing a crystalline form of a salt of the compound of formula (I), comprising: mixing one type of crystalline form of the salt of the compound of formula (I) with ethyl acetate and water and heating and stirring until the solution becomes clear, cooling for crystallization, and then filtering and drying under reduced pressure to give another type of crystalline form of the salt of the compound of formula (I).

In yet another aspect, the present application provides a crystalline composition comprising the crystalline form of the compound of formula (I), the crystalline form I or the crystalline form II of the hydrochloride of the compound of formula (I), the crystalline form of the fumarate of the compound of formula (I), the crystalline form of the maleate of the compound of formula (I), the crystalline form I of the methanesulfonate of the compound of formula (I), the crystalline form II of the methanesulfonate of the compound of formula (I), or the crystalline form of the hydrobromide of the compound of formula (I), wherein the crystalline form makes up 50% or more, preferably 80% or more, more preferably 90% or more, and most preferably 95% or more, by weight, of the crystalline composition.

In yet another aspect, the present application provides a pharmaceutical composition comprising a therapeutically or prophylactically effective amount of the crystalline form of the compound of formula (I), the hydrochloride of the compound of formula (I) or the crystalline form I or the crystalline form II thereof, the fumarate of the compound of formula (I) or the crystalline form thereof, the maleate of the compound of formula (I) or the crystalline form thereof, the methanesulfonate of the compound of formula (I) or the crystalline form I or the crystalline form II thereof, the hydrobromide of the compound of formula (I) or the crystalline form thereof, or the crystalline composition described above disclosed herein. The pharmaceutical composition disclosed herein may or may not contain a pharmaceutically acceptable excipient. In addition, the pharmaceutical composition disclosed herein may further comprise one or more additional therapeutic agents.

In yet another aspect, the present application provides a method for treating or preventing an ER-related disease, comprising administering to a subject in need thereof a therapeutically or prophylactically effective amount of the crystalline form of the compound of formula (I), the hydrochloride of the compound of formula (I) or the crystalline form I or the crystalline form II thereof, the fumarate of the compound of formula (I) or the crystalline form thereof, the maleate of the compound of formula (I) or the crystalline form thereof, the methanesulfonate of the compound of formula (I) or the crystalline form I or the crystalline form II thereof, the hydrobromide of the compound of formula (I) or the crystalline form thereof, or the crystalline composition described above, or the pharmaceutical composition described above.

In yet another aspect, the present application provides use of the crystalline form of the compound of formula (I), the hydrochloride of the compound of formula (I) or the crystalline form I or the crystalline form II thereof, the fumarate of the compound of formula (I) or the crystalline form thereof, the maleate of the compound of formula (I) or the crystalline

form thereof, the methanesulfonate of the compound of formula (I) or the crystalline form I or the crystalline form II thereof, the hydrobromide of the compound of formula (I) or the crystalline form thereof, or the crystalline composition described above, or the pharmaceutical composition described above in the manufacture of a medicament for treating or preventing an ER-related disease.

In yet another aspect, the present application provides use of the crystalline form of the compound of formula (I), the hydrochloride of the compound of formula (I) or the crystalline form I or the crystalline form II thereof, the fumarate of the compound of formula (I) or the crystalline form thereof, the maleate of the compound of formula (I) or the crystalline form thereof, the methanesulfonate of the compound of formula (I) or the crystalline form I or the crystalline form II thereof, the hydrobromide of the compound of formula (I) or the crystalline form thereof, or the crystalline composition described above, or the pharmaceutical composition described above in treating or preventing an ER-related disease.

In yet another aspect, the present application provides the crystalline form of the compound of formula (I), the hydrochloride of the compound of formula (I) or the crystalline form I or the crystalline form II thereof, the fumarate of the compound of formula (I) or the crystalline form thereof, the maleate of the compound of formula (I) or the crystalline form thereof, the methanesulfonate of the compound of formula (I) or the crystalline form I or the crystalline form II thereof, the hydrobromide of the compound of formula (I) or the crystalline form thereof, or the crystalline composition described above, or the pharmaceutical composition described above for use in treating or preventing an ER-related disease.

In some embodiments of the present application, the ER-related disease is breast cancer.

In some embodiments of the present application, the ER-related disease is ER-positive breast cancer.

## Technical Effects

The crystalline forms of the compound and the salts thereof disclosed herein are easy to prepare, and have good solubility, physical stability, and chemical stability. In addition, they demonstrate good exposure via oral administration and good pharmacokinetic properties and are suitable for use as medicaments. The crystalline forms of the compound and the salts thereof disclosed herein have good chemical stability under acidic, basic and oxidative conditions and therefore are easy to store, and the instability of the medicine caused by excipients in a formulation is more easily avoided, which favors formula screening. The crystalline forms of the compound and the salts thereof disclosed herein have low hygroscopicity. For example, it can be seen from the DVS patterns that both the crystalline form I and the crystalline form II of the hydrochloride of the compound of formula (I) disclosed herein have lower hygroscopicity than the amorphous form of the monohydrochloride of the compound of formula (I), and particularly, the performance of the crystalline form I of the hydrochloride of the compound of formula (I) is more excellent. Each of the crystalline forms disclosed herein can have good properties in pharmacokinetics, bioavailability, hygroscopicity, fluidity, stability, solubility, purity, mass uniformity, and the like.

## Definitions and Description

Unless otherwise stated, the following terms and phrases used herein are intended to have the following meanings. A particular phrase or term, unless otherwise specifically defined, should not be considered uncertain or unclear, but construed according to its common meaning. When referring to a trade name, it is intended to refer to its corresponding commercial product or its active ingredient.

For any given crystalline form, the relative intensities of diffraction peaks may vary due to preferred orientations resulting from, e.g., crystalline morphology, as is well known in the field of crystallography. The peak intensity varies at a place where there is preferred orientation effect, while it is impossible for the diffraction peak position of crystalline form to vary. In addition, there may be slight errors in the measurement of the peak positions for any given crystalline form, as is also well known in the field of crystallography. For example, the peak positions may shift due to temperature changes, sample movement, or calibration of the instrument when analyzing a sample, and the error in the measurement of $2\Theta$ is sometimes about $\pm 0.2$ degree, and therefore, it is well known to those skilled in the art that this error should be taken into account when determining each crystalline structure.

DSC measures the transition temperature when a crystalline form absorbs or releases heat due to a change in crystalline structure or melting of the crystalline form. For the same crystalline forms of the same compound, the thermal transition temperature and melting point errors in successive analyses are typically within about $\pm 3$ °C, and a given DSC peak or melting point of a compound, when referred to, means the DSC peak or melting point $\pm$ 3 °C. DSC provides an auxiliary method to identify different crystalline forms. Different crystalline morphologies can be identified by their different transition temperatures. It should be noted that for a mixture, its DSC peak or melting point may vary over a larger range. Furthermore, the melting temperature is related to the heating rate due to decomposition in the melting process of a substance.

The "pharmaceutically acceptable excipient" refers to an inert substance administered with an active ingredient

to facilitate administration of the active ingredient, including but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration, PRC.

The term "crystalline composition" refers to a mixture consisting of one or more of the crystalline forms of the compound of formula (I), or formula (II), or formula (III), or formula (IV), or formula (V), or formula (VI) disclosed herein and other crystalline forms or amorphous forms of the compound, or other impurities. For example, a crystalline composition of the compound of formula (I) refers to a mixture comprising, in addition to a crystalline form of the compound of formula (I) disclosed herein, other crystalline forms or amorphous forms of the compound of formula (I), or other impurities.

The term "pharmaceutical composition" refers to a mixture consisting of one or more of the compounds, the salts thereof, or the crystalline forms thereof disclosed herein and optionally a pharmaceutically acceptable excipient. The pharmaceutical composition is intended to facilitate the administration of the compound of the present application to an organic entity.

Therapeutic dosages of the compound of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration.

The term "treating" or "treatment" means administering the compound or formulation described herein to ameliorate or eliminate a disease or one or more symptoms associated with the disease, and includes:

(i) inhibiting a disease or disease state, i.e., arresting its progression; and
(ii) alleviating a disease or disease state, i.e., causing its regression.

The term "preventing" means administering the compound or formulation described herein to prevent one or more symptoms associated with the disease, and includes: preventing the occurrence of the disease or disease state in a mammal, particularly when such a mammal is predisposed to the disease state but has not yet been diagnosed as having it.

The therapeutically effective amount of a crystalline form of the present application is from about 0.0001 to 20 mg/kg body weight (bw)/day, for example from 0.001 to 10 mg/kg bw/day.

The term "therapeutically or prophylactically effective amount" refers to an amount of the compound disclosed herein for (i) treating or preventing a specific disease, condition or disorder; (ii) alleviating, relieving or eliminating one or more symptoms of a specific disease, condition or disorder, or (iii) preventing or delaying onset of one or more symptoms of a specific disease, condition or disorder described herein. The amount of the compound disclosed herein that is considered as the "therapeutically effective amount" varies depending on the compound, the disease state and its severity, the administration regimen, and the age of the mammal to be treated, but can be determined routinely by those skilled in the art in accordance with their knowledge and the present disclosure.

Unless otherwise required, the word "comprise" and variations thereof such as "comprises" and "comprising" and equivalents thereof, used in the specification and the claims which follow, should be understood in an open-ended and non-exclusive sense, i.e., "including, but not limited to".

"One embodiment", "an embodiment", "in another embodiment" or "in some embodiments" used in the specification means that a specific reference element, structure or feature described in connection with the embodiment is included in at least one embodiment. Thus, the phrases "in one embodiment", "in an embodiment", "in another embodiment" and "in some embodiments" in various places throughout the specification are not necessarily all referring to the same embodiment. Furthermore, the specific elements, structures, or features may be combined in any suitable manner in one or more embodiments.

It should be understood that, unless otherwise specified clearly, the singular forms "a", "an", and "the" used in the specification and the appended claims of the present application include plural referents. In other words, unless otherwise specified clearly herein, singular terms encompass plural referents, and vice versa. Thus, for example, the mentioned reaction including "a catalyst" includes one catalyst, or two or more catalysts. It should be understood that, unless otherwise specified clearly, the term "or" is generally used in its sense including "and/or".

Unless otherwise stated herein, parameter values representing amounts of ingredients or physicochemical properties or reaction conditions and the like are to be understood as being modified in all cases by the term "about". When the term "about" is used to describe the present application, the term "about" indicates that there is an error value; for example, it means varying within a range of $\pm$5%, such as $\pm$1% or $\pm$0.1%, of a particular value. The intermediate compounds disclosed herein can be prepared by a variety of synthetic methods well known to those skilled in the art, including the specific embodiments listed below, embodiments formed by combinations thereof with other chemical

synthetic methods, and equivalents thereof known to those skilled in the art. The preferred embodiments include, but are not limited to, the examples disclosed herein.

The chemical reactions of the embodiments of the present application are carried out in a proper solvent that should be suitable for the chemical changes in the present application and the reagents and materials required. In order to acquire the compounds of the present application, it is sometimes necessary for those skilled in the art to modify or select a synthesis procedure or a reaction scheme based on the existing embodiments.

The present application is described in detail below by way of examples, which are not intended to limit the present application in any way.

All solvents used in the present application are commercially available and can be used without further purification.

The solvents used in the present application are commercially available.

The following abbreviations are used in the present application:

$N_2$: nitrogen; RH: relative humidity; mL: milliliter; L: liter; min: minute; °C: degree Celsius; μm: micrometer; mm: millimeter; μL: microliter; moL/L: mole per liter; s: second; nm: nanometer; MPa: megapascal; lux: lux; μw/cm$^2$: microwatt per square centimeter; h: hour; kg: kilogram; nM: nanomole; RRT: relative retention time; rpm: rotation speed.

The compounds disclosed herein are named according to conventional nomenclature rules in the art or using ChemDraw® software, and supplier's catalog names are given for commercially available compounds.

All patents, patent applications and other identified publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

Instruments and analytical methods

1.1. X-ray powder diffractometer (XRPD) method of the present application

Instrument model: PANalytical X'Pert$^3$ X-ray diffractometer

Method: taking a sample of about 10 mg for XRPD analysis.

X-ray type: Cu, Kα, Kα1 (Å): 1.540598; Kα2 (Å): 1.544426; intensity ratio Kα2/Kα1: 0.50; voltage: 45 kilovolts (kV); current: 40 milliamperes (mA); divergent slit: 1/16 degrees; scan mode: continuous; scan range: from 3.0 to 40.0 degrees.

1.2. Differential scanning calorimeter (DSC) method of the present application

Instrument model: TA Instruments Discovery DSC 2500 and Q200 differential scanning calorimeter

Method: taking a sample of 1-5 mg and placing it in a covered aluminum crucible, and heating the sample from room temperature to 350 °C at a heating rate of 10 °C/min in an atmosphere of 50 mL/min of dry $N_2$ while simultaneously recording the heat change of the sample in the heating process with TA software.

**1.3. Thermal gravimetric analyzer (TGA) method of the present application**

Instrument model: TA Instruments Q5000 and Discovery TGA 5500 thermogravimetric analyzer

Method: taking a sample of 2-5 mg and placing it in a platinum crucible, adopting a sectional high-resolution detection mode, and heating the sample from room temperature to 350 °C at a heating rate of 10 °C/min in an atmosphere of 50 mL/min of dry $N_2$ while simultaneously recording the heat change of the sample in the heating process with TA software.

**1.4. Dynamic vapor sorption (DVS) method of the present application**

Instrument model: DVS Intrinsic instrument from SMS (Surface Measurement Systems) Inc.
DVS test parameters:
Temperature: 25 °C; sample amount: 10-30 mg; protective gas and flow: $N_2$, 200 mL/min; dm/dt: 0.002%/min; minimum dm/dt equilibration time: 10 min; maximum equilibration time: 180 min; RH range: 0% RH-95% RH-0% RH; RH gradient: 10% (90% RH-0% RH-90% RH), 5% (95% RH-90% RH and 90% RH-95% RH).

**1.5. Method of determining chloride ion** content **of the present application**

Instrument model: LC-20AD sp instrument from SHIMADZU Inc.
Software type: Lab Solution Version 5.92
Chromatographic column and mobile phase: SHIMADZU Shim-pack IC-A3 4.6 mm × 15 cm 5 μm; 8.0 mmol/L of p-hydroxybenzoic acid + 3.2 mmol/L Bis-Tris buffer
Flow rate of mobile phase: 1.5 mL/min; column's internal temperature: 40 °C
Method: the external standard single-point method; precisely weighing out a test sample and a reference sample to prepare aqueous solutions, precisely measuring out a certain amount of each of the solutions for injection, recording chromatograms, measuring peak areas (or peak heights) of the test substances in the reference sample solution and the test sample solution, and calculating their contents according to the following formula:

$$\text{Content } (c_x) = c_R \times A_x/A_R$$

wherein, $c_R$: the concentration content of the reference sample; $A_x$: the peak area of the test sample; $A_R$: the peak area of the reference sample.

**BRIEF DESCRIPTION OF THE DRAWINGS**

FIG. 1 is an XRPD pattern of the crystalline form of the compound of formula (I).
FIG. 2 is a DSC pattern of the crystalline form of the compound of formula (I).
FIG. 3 is a TGA pattern of the crystalline form of the compound of formula (I).
FIG. 4 is an XRPD pattern of the crystalline form I of the compound of formula (II).
FIG. 5 is a DSC and TGA pattern of the crystalline form I of the compound of formula (II).
FIG. 6 is a DVS pattern of the crystalline form I of the compound of formula (II).
FIG. 7 is an XRPD pattern of the crystalline form of the compound of formula (III).
FIG. 8 is a DSC and TGA pattern of the crystalline form of the compound of formula (III).
FIG. 9 is an XRPD pattern of the crystalline form of the compound of formula (IV).
FIG. 10 is a DSC and TGA pattern of the crystalline form of the compound of formula (IV).
FIG. 11 is an XRPD pattern of the crystalline form I of the compound of formula (V).
FIG. 12 is an XRPD pattern of the crystalline form II of the compound of formula (V).
FIG. 13 is an XRPD pattern of the crystalline form of the compound of formula (VI).
FIG. 14 is an XRPD pattern of the monohydrochloride of the compound of formula (I) of Example 1.
FIG. 15 is a DSC and TGA pattern of the monohydrochloride of the compound of formula (I) of Example 1.
FIG. 16 is a DVS pattern of the monohydrochloride of the compound of formula (I) of Example 1.
FIG. 17 is an XRPD pattern of the crystalline form II of the compound of formula (II).
FIG. 18 is a DSC and TGA pattern of the crystalline form II of the compound of formula (II).
FIG. 19 is a DVS pattern of the crystalline form II of the compound of formula (II).

**DETAILED DESCRIPTION**

The present application is described in detail below by way of examples, which, however, are not intended to disadvantageously limit the scope of the present application in any way. The compounds of the present application can

be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application. It will be apparent to those skilled in the art that various changes and modifications can be made to the specific embodiments without departing from the spirit and scope of the present application, and these changes and modifications do not depart from the protection scope of the present application.

**Example 1: Preparation of Compound of Formula (I) and Monohydrochloride thereof**

With reference to the method disclosed in Example 3 of WO2020125640, the compound of formula (I) and the monohydrochloride of the compound of formula (I) were obtained.

The monohydrochloride of the compound of formula (I) is an amorphous form, and its XRPD, DSC, TGA and DVS patterns are shown in FIGs. 14-16. The DSC pattern shows an endothermic peak at 80.0 °C and the TGA pattern shows a weight loss of 3.94% at 120.0 °C.

**Example 2: Preparation of Crystalline Form of Compound of Formula (I)**

53 g of the compound of formula (I) was weighed out and placed in a 1L transparent glass bottle, and 500 mL of ethyl acetate was added. The mixture was heated to 100 °C and stirred for 20 min until the system became clear. Heating was stopped. The mixture was naturally cooled to 25 °C and then stirred for 11 h and 40 min at 25 °C. The resulting mixture was filtered, and the filter cake was dried under reduced pressure (45 °C, no more than -0.1 MPa) to give the crystalline form of the compound of formula (I). Its XRPD, DSC and TGA patterns are shown in FIGs. 1-3. 1H NMR (400 MHz, DMSO-$d_6$) δ = 11.46 (s, 1H), 7.62 (d, *J* = 2.4 Hz, 1H), 7.49 (d, *J* = 7.6Hz, 1H), 7.38 (d, *J* = 8.0 Hz, 1H), 7.33 - 7.27 (m, 2H), 7.25 - 7.08 (m, 6H), 6.66 - 6.53 (m, 2H), 6.51 - 6.44 (m, 1H), 4.14 (t, *J* = 5.6 Hz, 2H), 3.29 (d, *J* = 4.8 Hz, 2H), 2.97 (s, 3H), 2.83 (s, 3H), 2.75 (t, *J* = 5.2 Hz, 2H), 2.48 - 2.41 (m, 2H), 2.02 (br s, 1H), 0.89 (t, J = 7.6 Hz, 3H).

**Example 3: Preparation of Crystalline Form I of Compound of Formula (II)**

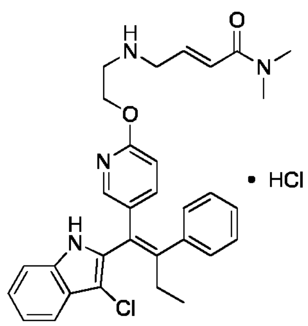

(II)

The crystalline form of formula (I) prepared in Example 2 (57.5 g) and 690 mL of ethyl acetate were added sequentially to a glass bottle and heated to 90 °C. The system became clear. Then a solution of 9.5 mL of concentrated hydrochloric acid (36-38 wt%, 1.05 eq) in ethyl acetate (20 mL) was added, and the mixture was stirred at 90 °C for 17 h, cooled to room temperature, and then filtered. The filter cake was rinsed twice with ethyl acetate (20 mL) and dried under reduced pressure (50 °C, no more than -0.1 MPa) to give the crystalline form I of the compound of formula (II). Its XRPD, DSC, TGA and DVS patterns are shown in FIGs. 4-6. Analysis showed that the average content of Cl ions was 6.19%; the salt formation number was determined to be 1. $^1$H NMR (400 MHz, DMSO-$d_6$) δ ppm 11.55 (s, 1H), 9.31 (br s, 2H), 7.66 (d, *J* = 2.0 Hz, 1H), 7.49 (d, *J* = 8.0 Hz, 1H), 7.39 (d, *J* = 8.0 Hz, 1H), 7.10-7.33 (m, 8H), 6.81 (d, *J*= 15.2 Hz, 1H), 6.63 (d, *J*= 8.6 Hz, 1H), 6.54-6.61 (m, 1H), 4.37 (t, *J*= 4.8 Hz, 2H), 3.76 (d, *J*= 6.4 Hz, 2H), 3.17-3.28 (m, 2H), 3.02 (s, 3H), 2.86 (s, 3H), 2.41-2.48 (m, 2H), 0.89 (t, *J* = 7.6 Hz, 3H).

**Example 4: Preparation of Crystalline Form of Compound of Formula (III)**

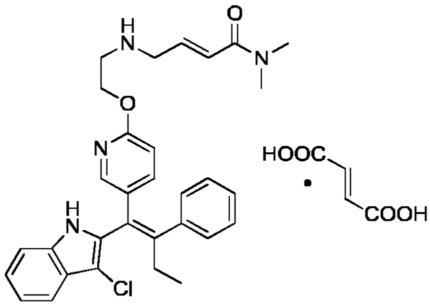

(III)

The crystalline form of the compound of formula (I) prepared in Example 2 (101.34 mg) was weighed out and added to an 8mL transparent glass bottle, and 1.4 mL of acetone was added. The mixture was heated at 53 °C until the system became clear. Fumaric acid (23.83 mg, 1.05 eq) was added to the system. The mixture was stirred at 53 °C for 0.5 h and then cooled to room temperature and stirred for 12 h. The resulting mixture was filtered, and the filter cake was dried under reduced pressure (45 °C, no more than -0.1 MPa) to give the crystalline form of the compound of formula (III). Its XRPD, DSC and TGA patterns are shown in FIGs. 7-8. 1H NMR (400 MHz, DMSO-$d_6$) δ ppm 11.49 (br s, 1H), 7.59-7.66 (m, 1H), 7.49 (d, *J* = 7.6 Hz, 1H), 7.38 (d, *J* = 7.6 Hz, 1H), 7.10-7.33 (m, 8H), 6.49-6.63 (m, 5H), 4.21 (d, *J*= 4.4 Hz, 2H), 3.45(s, 2H), 2.98 (s, 3H), 2.92 (s, 2H), 2.84 (s, 3H), 2.44 (m, 2H), 0.89 (t, *J* = 7.4 Hz, 3H).

**Example 5: Preparation of Crystalline Form of Compound of Formula (IV)**

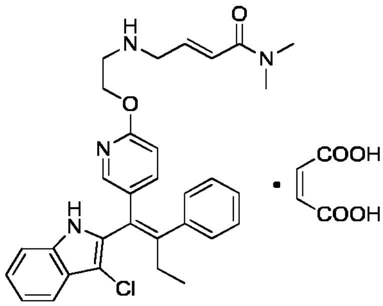

(IV)

The crystalline form of the compound of formula (I) prepared in Example 2 (100.45 mg) was weighed out and added to an 8mL glass bottle, and 1.2 mL of ethyl acetate was added. The mixture was heated to 80 °C for dissolution. Then maleic acid (1.05 eq, 23.84 mg) was added. The mixture was stirred at 78 °C for 1 h. Heating was then stopped. The mixture was naturally cooled to room temperature and stirred for another 12 h at room temperature. The resulting mixture was filtered, and the filter cake was dried under reduced pressure (45 °C, no more than -0.1 MPa) to give the crystalline form of the compound of formula (IV). Its XRPD, DSC and TGA patterns are shown in FIGs. 9-10. 1H NMR (400 MHz, DMSO-$d_6$) δ ppm 11.50 (s, 1H), 7.66 (d, *J* = 2.4 Hz, 1H), 7.49 (d, *J* = 7.6 Hz, 1H), 7.39 (d, *J* = 8.0 Hz, 1H), 7.11-7.33 (m, 8H), 6.77 (d, *J* = 14.8 Hz, 1H), 6.50-6.67 (m, 2H), 6.02 (s, 2H), 4.33 (t, *J*= 4.8 Hz, 2H), 3.77 (d, *J*= 6.4 Hz, 2H), 3.25 (t, *J*= 4.8 Hz, 2H), 3.02 (s, 3H), 2.86 (s, 3 H), 2.42-2.48 (m, 2H), 0.89 ppm (t, *J* = 7.4 Hz, 3H).

**Example 6: Preparation of Crystalline Form I of Compound of Formula (V)**

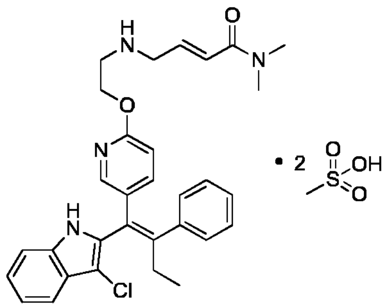

(V)

The crystalline form of the compound of formula (I) prepared in Example 2 (100.25 mg) was weighed out and added to an 8mL glass bottle, and 1.4 mL of ethyl acetate was added. The mixture was heated to 80 °C for dissolution. Then methanesulfonic acid (1.05 eq, 14.5 μL) was added. The mixture was stirred at 78 °C for 1 h. Heating was then stopped. The mixture was naturally cooled to room temperature and stirred for another 12 h at room temperature. The resulting mixture was filtered, and the filter cake was dried under reduced pressure (45 °C, no more than -0.1 MPa) to give the crystalline form I of the compound of formula (V). Its XRPD pattern is shown in FIG. 11. 1H NMR (400 MHz, DMSO-$d_6$) δ ppm 11.51 (s, 1H), 8.87 (br s, 2H), 7.66 (d, *J* = 2.0 Hz, 1H), 7.49 (d, *J* = 7.6 Hz, 1H), 7.39 (d, *J* = 8.0 Hz, 1H), 7.27-7.35 (m, 2H), 7.16-7.27 (m, 5H), 7.07-7.16 (m, 1H), 6.79 (d, *J* = 15.2 Hz, 1H), 6.64 (d, *J* = 8.4 Hz, 1H), 6.54 (m, 1H), 4.35 (t, *J* = 4.8 Hz, 2H), 3.79 (q, *J* = 5.6 Hz, 2H), 3.26 (br s, 2H), 3.02 (s, 3H), 2.86 (s, 3H), 2.41-2.47 (m, 2H), 2.36 (s, 6H), 0.89 ppm (t, *J* = 7.4 Hz, 3H).

**Example 7: Preparation of Crystalline Form II of Compound of Formula (V)**

The crystalline form of the compound of formula (I) prepared in Example 2 (100.39 mg) was weighed out and added to an 8mL glass bottle, and 1.4 mL of acetone was added. The mixture was heated to 53 °C for dissolution. Then methanesulfonic acid (1.05 eq, 14.5 μL) was added. The mixture was stirred at 53 °C for 0.5 h. Heating was then stopped. The mixture was naturally cooled to room temperature and stirred for another 12 h at room temperature. The resulting mixture was filtered, and the filter cake was dried under reduced pressure (50 °C) to give the crystalline form II of the compound of formula (V). Its XRPD pattern is shown in FIG. 12. 1H NMR (400 MHz, DMSO-$d_6$) δ ppm 11.52 (s, 1H), 8.88 (br s, 2H), 7.66 (d, *J* = 2.4 Hz, 1H), 7.49 (d, *J* = 8.0 Hz, 1H), 7.40 (d, *J* = 8.0 Hz, 1H), 7.27-7.35 (m, 2H), 7.16-7.27 (m, 5H), 7.08-7.16 (m, 1H), 6.79 (d, *J* = 15.2 Hz, 1H), 6.64 (d, *J* = 8.8 Hz, 1H), 6.54 (m, 1H), 4.34 (t, *J* = 5.2 Hz, 2H), 3.79 (q, J = 5.6 Hz, 2H), 3.26 (br s, 2H), 3.02 (s, 3H), 2.86 (s, 3H), 2.42-2.48 (m, 2H), 2.37 (s, 6H), 0.89 (t, *J* = 7.4 Hz, 3H).

**Example 8: Preparation of Crystalline Form of Compound of Formula (VI)**

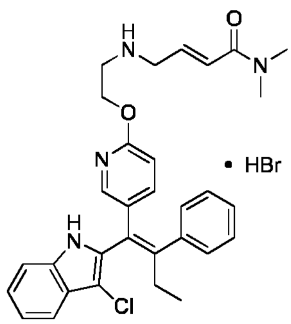

(VI)

The crystalline form of the compound of formula (I) prepared in Example 2 (about 1 g) was weighed out and added to a glass bottle, and 14 mL of acetone was added. The mixture was heated to 53 °C for dissolution. Then hydrobromic acid (48%w aqueous solution, 1.05 eq, 225 μL) was added. The mixture was stirred at 53 °C for 0.5 h. Heating was then stopped. The mixture was naturally cooled to room temperature and stirred for another 12 h at room temperature. The resulting mixture was filtered, and the filter cake was dried under reduced pressure (50 °C) to give the crystalline form of the compound of formula (VI). Its XRPD pattern is shown in FIG. 13. $^1H$ NMR (400 MHz, DMSO-$d_6$)

δ ppm 11.47-11.61 (m, 1H), 8.94-9.23 (m, 2H), 7.66 (d, *J* = 2.0 Hz, 1H), 7.49 (d, *J* = 7.6 Hz, 1H), 7.39 (d, *J*= 8.0 Hz, 1H), 7.27-7.33 (m, 2H), 7.16-7.27 (m, 5H), 7.09-7.16 (m, 1H), 6.76-6.89 (m, 1H), 6.64 (d, *J*=8.4 Hz, 1H), 6.51-6.61 (m, 1H), 4.37 (br s, 2H), 3.78 (d, *J* = 6.4 Hz, 2H), 3.19-3.29 (m, 2H), 3.02 (s, 3H), 2.86 (s, 3H), 2.41-2.48 (m, 2H), 0.89 (t, *J* = 7.4 Hz, 3H).

**Example 9: Preparation of Crystalline Form II of Compound of Formula (II)**

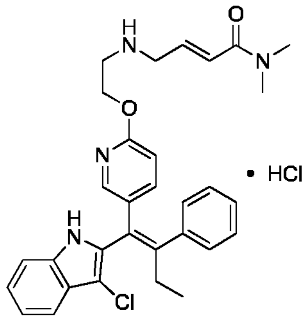

(II)

The crystalline form I of the compound of formula (II) (1 g), 3 mL of ethyl acetate, and 3 mL of water were added sequentially to a glass bottle. The mixture was heated in an oil bath to 60 °C and stirred for 0.1 h. The system became clear. The oil bath was replaced with a 20 °C water bath for cooling the system to room temperature, and the mixture was stirred for 12 h at room temperature and filtered. The filter cake was dried under reduced pressure (50 °C, no more than -0.1 MPa) to give the crystalline form II of the compound of formula (II). Its XRPD, DSC, TGA and DVS patterns are shown in FIGs. 17-19. 1H NMR (400 MHz, DMSO-$d_6$) δ = 11.58 (s, 1H), 9.49 (s, 2H), 7.67 (d, *J* = 2.4 Hz, 1H), 7.49 (d, *J* = 8.0 Hz, 1H), 7.40 (d, *J* = 8.0 Hz, 1H), 7.34 - 7.28 (m, 2H), 7.26 - 7.11 (m, 6H), 6.83 (d, *J* = 15.2 Hz, 1H), 6.67 - 6.53 (m, 2H), 4.40 (t, J = 4.8 Hz, 2H), 3.76 (d, *J* = 6.4 Hz, 2H), 3.23 (t, *J* = 4.8 Hz, 2H), 3.03 (s, 3H), 2.87 (s, 3H), 2.49 - 2.41 (m, 2H), 0.90 (t, *J* = 7.2 Hz, 3H)

**Experimental Example 1: Study on Properties of Crystalline Forms**

1-2 mg of a test sample was taken and placed in a semi-automatic filter vial, and 450 μL of purified water or FeSSIF simulated fluid (fed state simulated intestinal fluid) was added to give a supersaturated suspension. The sample was vortexed for at least 2 min. The vial was placed on a plate shaker and shaken at 37 °C at 800 rpm for 24 h. After centrifugal filtration, the sample was subjected to HPLC-UV linear quantitative analysis for concentration.

FeSSIF (fed state simulated intestinal fluid): an aqueous buffer solution comprising 0.282% (w/v) lecithin, 0.806% (w/v) sodium taurocholate, 0.865% (w/v) acetic acid, 1.52% (w/v) potassium chloride, with a pH of 5.0±0.05.

**Table 8**

| Test compound | Vehicle | Solubility (mg/mL) |
|---|---|---|
| Crystalline form of compound of formula (I) | Water | <0.0008 |
| Crystalline form I of compound of formula (II) | Water | 0.237 |
| Crystalline form of compound of formula (I) | FeSSIF | 0.316 |
| Crystalline form I of compound of formula (II) | FeSSIF | 1.901 |
| Crystalline form of compound of formula (III) | FeSSIF | 1.946 |
| Crystalline form of compound of formula (VI) | FeSSIF | 1.368 |

**Experimental Example 2: *In Vivo* Pharmacokinetic Study of Crystalline Form I of Compound of Formula (II)**

**Experimental objective:**

This experiment was intended to evaluate the pharmacokinetic behavior of the compound after a single intragastric administration and to investigate bioavailability after intragastric administration.

**Procedures:**

Two female beagle dogs were each given a single oral dose of the crystalline form I of the compound of formula (II) at 100 mg/kg at 1 h after feeding, and plasma samples were taken at 0.0833 (5 min), 0.25 (15 min), 0.5 (30 min), 1, 2, 4, 6, 8, 12, and 24 h after administration and assayed for the concentration of the test substance by LC-MS/MS. The results are shown in Table 9.

**Table 9. Evaluation results of *in vivo* PK properties**

| Pharmacokinetic parameter | Mean |
|---|---|
| $C_{max}$ (nM) | 14000 |
| $T_{max}$ (h) | 6.00 |
| $T_{1/2}$ (h) | 14.9 |
| $AUC_{0\text{-}last}$ (nM•h) | 228745 |
| $T_{1/2}$: half-life; $AUC_{0\text{-}last}$: area under the curve; $C_0$: initial concentration; $C_{max}$: maximum concentration; $T_{max}$: time to reach maximum concentration. | |

**Experimental Example 3: Forced Degradation Experiment**

Control group: 20 mg of a sample was weighed out, a proper amount of diluent (acetonitrile: water = 1:1, v/v) was added to dissolve the sample, and then the solution was brought to a volume of 100 mL with the diluent (acetonitrile: water = 1:1, v/v). A proper amount of the solution was taken for analysis.

Acidic degradation: 20 mg of the sample was weighed out, and 1 mL of a 1 M aqueous HCl solution was added. After sitting at room temperature for 24 h, the mixture was neutralized with 1 mL of a 1 M aqueous NaOH solution. The solution was brought to a volume of 100 mL with the diluent (acetonitrile: water = 1:1, v/v). A proper amount of the solution was taken for analysis.

Basic degradation: 20 mg of the sample was weighed out, and 1 mL of a 1 M aqueous NaOH solution was added. After sitting at room temperature for 24 h, the mixture was neutralized with 1 mL of a 1 M aqueous HCl solution. The solution was brought to a volume of 100 mL with the diluent (acetonitrile: water = 1:1, v/v). A proper amount of the solution was taken for analysis.

Oxidative degradation: 20 mg of the sample was weighed out, 1 mL of a 3% aqueous hydrogen peroxide solution was added, and the mixture was left at room temperature for 24 h. The solution was brought to a volume of 100 mL with the diluent (acetonitrile: water = 1:1, v/v). A proper amount of the solution was taken for analysis.

The experimental results are shown in Tables 10-11.

**Table 10. Forced degradation results of the crystalline form I of the compound of formula (II)**

| **Degradation type** | Not degraded | Acidic | Basic | Oxidative |
|---|---|---|---|---|
| **Total impurities (%)** | 0.34 | 0.34 | 0.35 | 0.92 |

**Table 11. Forced degradation results of the monohydrochloride of the compound of formula (I) (amorphous form)**

| **Degradation type** | Not degraded | Acidic | Basic | Oxidative |
|---|---|---|---|---|
| **Total impurities (%)** | 0.60 | 0.56 | 0.54 | 3.5 |

Therefore, the crystalline forms disclosed herein can exhibit good solubility, hygroscopicity, pharmacokinetic

properties, bioavailability, stability, and the like.

## Claims

1. A crystalline form of a compound of formula (I), a salt of the compound of formula (I) or a crystalline form thereof, wherein the salt of the compound of formula (I) is selected from the group consisting of a fumarate, a maleate, a methanesulfonate, and a hydrobromide, and the crystalline form of the salt of the compound of formula (I) is selected from the group consisting of a crystalline form of the hydrochloride, a crystalline form of the fumarate, a crystalline form of the maleate, a crystalline form of the methanesulfonate, and a crystalline form of the hydrobromide,

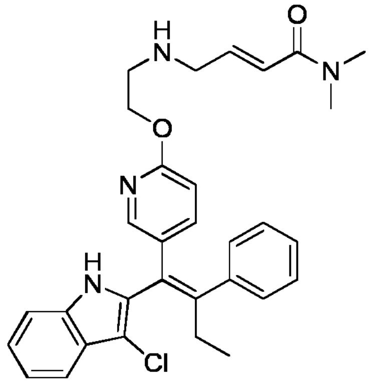

(I)

2. The crystalline form of the compound of formula (I), the salt of the compound of formula (I) or the crystalline form thereof according to claim 1, wherein the crystalline form of the compound of formula (I) comprises 3, 4, 5, 6, 7, or 8 diffraction peaks in an X-ray powder diffraction pattern using Cu Kα radiation at 2Θ values selected from the group consisting of: 13.14±0.20°, 16.62±0.20°, 17.60±0.20°, 19.98±0.20°, 21.78±0.20°, 22.38±0.20°, 23.41±0.20°, and 24.22±0.20°;

   or the crystalline form of the compound of formula (I) has diffraction peaks in an X-ray powder diffraction pattern using Cu Kα radiation at 2Θ values of 17.60±0.20°, 19.98±0.20°, and 23.41±0.20°;
   or the crystalline form of the compound of formula (I) has diffraction peaks in an X-ray powder diffraction pattern using Cu Kα radiation at 2Θ values of 13.14±0.20°, 16.62±0.20°, 17.60±0.20°, 19.98±0.20°, 21.78±0.20°, 22.38±0.20°, 23.41±0.20°, and 24.22±0.20°;
   or the crystalline form of the compound of formula (I) has diffraction peaks in an X-ray powder diffraction pattern using Cu Kα radiation at 2Θ values of 13.14±0.20°, 14.68±0.20°, 16.62±0.20°, 17.60±0.20°, 19.98±0.20°, 21.32±0.20°, 21.78±0.20°, 22.38±0.20°, 23.41±0.20°, 24.22±0.20°, 26.46±0.20°, and 28.84±0.20°;
   or the crystalline form of the compound of formula (I) has diffraction peaks in an X-ray powder diffraction pattern using Cu Kα radiation at 2Θ values of: 8.56±0.20°, 10.13±0.20°, 12.16±0.20°, 13.14±0.20°, 13.54±0.20°, 14.68±0.20°, 15.68±0.20°, 16.38±0.20°, 16.62±0.20°, 17.24±0.20°, 17.60±0.20°, 18.86±0.20°, 19.22±0.20°, 19.46±0.20°, 19.98±0.20°, 20.89±0.20°, 21.32±0.20°, 21.78±0.20°, 22.38±0.20°, 22.70±0.20°, 23.08±0.20°, 23.41±0.20°, 23.70±0.20°, 24.01±0.20°, 24.22±0.20°, 24.62±0.20°, 24.89±0.20°, 25.26±0.20°, 25.92±0.20°, 26.46±0.20°, 26.92±0.20°, 27.32±0.20°, 28.18±0.20°, 28.54±0.20°, 28.84±0.20°, 29.42±0.20°, 30.24±0.20°, 30.70±0.20°, 30.94±0.20°, 31.64±0.20°, 32.71±0.20°, 33.22±0.20°, and 34.84±0.20°;
   or the crystalline form of the compound of formula (I) has an X-ray powder diffraction pattern using Cu Kα radiation as shown in FIG. 1.

3. The crystalline form of the compound of formula (I), the salt of the compound of formula (I) or the crystalline form thereof according to claim 1 or 2, wherein the crystalline form of the compound of formula (I) has a differential scanning calorimetry curve showing an endothermic peak at 144.92±3 °C.

4. The crystalline form of the compound of formula (I), the salt of the compound of formula (I) or the crystalline form thereof according to claim 1, wherein the crystalline form of the salt of the compound of formula (I) is the crystalline form of the hydrochloride, which comprises 3, 4, 5, 6, 7, or 8 diffraction peaks in an X-ray powder diffraction pattern

using Cu Kα radiation at 2Θ values selected from the group consisting of: 5.97±0.20°, 7.58±0.20°, 16.49±0.20°, 17.63±0.20°, 20.25±0.20°, 22.84±0.20°, 23.99±0.20°, and 24.62±0.20°;

or the crystalline form of the hydrochloride of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 5.97±0.20°, 7.58±0.20°, 11.08±0.20°, 11.38±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.99±0.20°, and 24.62±0.20°;
or the crystalline form of the hydrochloride of the compound of formula (I) comprises 3, 4, 5, 6, 7, 8, 9, 10, 11, 12 or more diffraction peaks in an X-ray powder diffraction pattern using Cu Kα radiation at 2Θ values selected from the group consisting of: 5.97±0.20°, 7.58±0.20°, 9.83±0.20°, 11.08±0.20°, 11.38±0.20°, 12.69±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.79±0.20°, 23.99±0.20°, and 24.62±0.20°;
or the crystalline form of the hydrochloride of the compound of formula (I) has diffraction peaks in an X-ray powder diffraction pattern using Cu Kα radiation at 2Θ values of 5.97±0.20°, 7.58±0.20°, and 17.63±0.20°;
or the crystalline form of the hydrochloride of the compound of formula (I) has diffraction peaks in an X-ray powder diffraction pattern using Cu Kα radiation at 2Θ values of 5.97±0.20°, 7.58±0.20°, 16.49±0.20°, 17.63±0.20°, 20.25±0.20°, 22.84±0.20°, 23.99±0.20°, and 24.62±0.20°;
or the crystalline form of the hydrochloride of the compound of formula (I) has diffraction peaks in an X-ray powder diffraction pattern using Cu Kα radiation at 2Θ values of 5.97±0.20°, 7.58±0.20°, 11.08±0.20°, 11.38±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.99±0.20°, and 24.62±0.20°;
or the crystalline form of the hydrochloride of the compound of formula (I) has diffraction peaks in an X-ray powder diffraction pattern using Cu Kα radiation at 2Θ values of 5.97±0.20°, 7.58±0.20°, 9.83±0.20°, 11.08±0.20°, 11.38±0.20°, 12.69±0.20°, 14.14±0.20°, 15.30±0.20°, 16.49±0.20°, 17.63±0.20°, 18.44±0.20°, 19.72±0.20°, 20.25±0.20°, 20.66±0.20°, 21.39±0.20°, 21.88±0.20°, 22.23±0.20°, 22.84±0.20°, 23.79±0.20°, 23.99±0.20°, and 24.62±0.20°;
or the crystalline form of the hydrochloride of the compound of formula (I) has an X-ray powder diffraction pattern using Cu Kα radiation as shown in FIG. 4;
or the crystalline form of the hydrochloride of the compound of formula (I) comprises 3, 4, 5, 6, 7, or 8 diffraction peaks in an X-ray powder diffraction pattern using Cu Kα radiation at 2Θ values selected from the group consisting of: 9.84±0.20°, 16.31±0.20°, 16.76±0.20°, 20.09±0.20°, 22.61±0.20°, 23.65±0.20°, 24.55±0.20°, and 25.32±0.20°;
or the crystalline form of the hydrochloride of the compound of formula (I) has diffraction peaks in an X-ray powder diffraction pattern using Cu Kα radiation at 2Θ values of 9.84±0.20°, 16.76±0.20°, and 20.09±0.20°;
or the crystalline form of the hydrochloride of the compound of formula (I) has diffraction peaks in an X-ray powder diffraction pattern using Cu Kα radiation at 2Θ values of 9.84±0.20°, 16.31±0.20°, 16.76±0.20°, 20.09±0.20°, 22.61±0.20°, 23.65±0.20°, 24.55±0.20°, and 25.32±0.20°;
or the crystalline form of the hydrochloride of the compound of formula (I) has diffraction peaks in an X-ray powder diffraction pattern using Cu Kα radiation at 2Θ values of 9.84±0.20°, 10.59±0.20°, 12.39±0.20°, 12.61±0.20°, 13.99±0.20°, 16.31±0.20°, 16.76±0.20°, 18.31±0.20°, 18.72±0.20°, 19.06±0.20°, 19.55±0.20°, 19.72±0.20°, 20.09±0.20°, 20.77±0.20°, 21.22±0.20°, 22.61±0.20°, 23.04±0.20°, 23.41±0.20°, 23.65±0.20°, 24.55±0.20°, 25.11±0.20°, 25.32±0.20°, 26.49±0.20°, 26.90±0.20°, 27.71±0.20°, 28.27±0.20°, 28.47±0.20°, 29.22±0.20°, 29.61±0.20°, 30.22±0.20°, 31.11±0.20°, 31.47±0.20°, and 34.18±0.20°;
or the crystalline form of the hydrochloride of the compound of formula (I) has an X-ray powder diffraction pattern using Cu Kα radiation as shown in FIG. 17.

**5.** The crystalline form of the compound of formula (I), the salt of the compound of formula (I) or the crystalline form thereof according to claim 4, wherein the crystalline form of the hydrochloride of the compound of formula (I) has a differential scanning calorimetry curve showing an endothermic peak at 204.5±3 °C; or the crystalline form of the hydrochloride of the compound of formula (I) has a differential scanning calorimetry curve showing endothermic peaks at 101.4±3°C, 113.3±3°C, and 195.9±3°C.

**6.** The crystalline form of the compound of formula (I), the salt of the compound of formula (I) or the crystalline form thereof according to claim 1, wherein the crystalline form of the salt of the compound of formula (I) is the crystalline

form of the fumarate, which comprises 3, 4, 5, 6, 7, or 8 diffraction peaks in an X-ray powder diffraction pattern using Cu Kα radiation at 2Θ values selected from the group consisting of: 5.12±0.20°, 9.20±0.20°, 14.99±0.20°, 18.08±0.20°, 19.17±0.20°, 21.39±0.20°, 22.57±0.20°, and 25.15±0.20°;

or the crystalline form of the fumarate of the compound of formula (I) has diffraction peaks in an X-ray powder diffraction pattern using Cu Kα radiation at 2Θ values of 5.12±0.20°, 14.99±0.20°, and 19.17±0.20°;
or the crystalline form of the fumarate of the compound of formula (I) has diffraction peaks in an X-ray powder diffraction pattern using Cu Kα radiation at 2Θ values of 5.12±0.20°, 9.20±0.20°, 14.99±0.20°, 18.08±0.20°, 19.17±0.20°, 21.39±0.20°, 22.57±0.20°, and 25.15±0.20°;
or the crystalline form of the fumarate of the compound of formula (I) has diffraction peaks in an X-ray powder diffraction pattern using Cu Kα radiation at 2Θ values of 5.12±0.20°, 9.20±0.20°, 12.03±0.20°, 14.99±0.20°, 15.31±0.20°, 16.75±0.20°, 17.62±0.20°, 18.08±0.20°, 18.83±0.20°, 19.17±0.20°, 20.80±0.20°, 21.39±0.20°, 22.21±0.20°, 22.57±0.20°, 23.09±0.20°, 23.50±0.20°, 24.42±0.20°, 25.15±0.20°, 25.78±0.20°, 27.14±0.20°, 28.25±0.20°, 29.54±0.20°, 30.50±0.20°, 31.09±0.20°, and 32.16±0.20°;
or the crystalline form of the fumarate of the compound of formula (I) has an X-ray powder diffraction pattern using Cu Kα radiation as shown in FIG. 7.

**7.** The crystalline form of the compound of formula (I), the salt of the compound of formula (I) or the crystalline form thereof according to claim 6, wherein the crystalline form of the fumarate of the compound of formula (I) has a differential scanning calorimetry curve showing an endothermic peak at 164.5±3 °C.

**8.** The crystalline form of the compound of formula (I), the salt of the compound of formula (I) or the crystalline form thereof according to claim 1, wherein the crystalline form of the salt of the compound of formula (I) is the crystalline form of the maleate, which comprises 3, 4, 5, 6, 7, or 8 diffraction peaks in an X-ray powder diffraction pattern using Cu Kα radiation at 2Θ values selected from the group consisting of: 4.55±0.20°, 12.85±0.20°, 16.19±0.20°, 16.68±0.20°, 18.12±0.20°, 21.18±0.20°, 22.71±0.20°, and 27.31±0.20°;

or the crystalline form of the maleate of the compound of formula (I) has diffraction peaks in an X-ray powder diffraction pattern using Cu Kα radiation at 2Θ values of 4.55±0.20°, 18.12±0.20°, and 21.18±0.20°;
or the crystalline form of the maleate of the compound of formula (I) has diffraction peaks in an X-ray powder diffraction pattern using Cu Kα radiation at 2Θ values of 4.55±0.20°, 12.85±0.20°, 16.19±0.20°, 16.68±0.20°, 18.12±0.20°, 21.18±0.20°, 22.71±0.20°, and 27.31±0.20°;
or the crystalline form of the maleate of the compound of formula (I) has diffraction peaks in an X-ray powder diffraction pattern using Cu Kα radiation at 2Θ values of 4.55±0.20°, 18.12±0.20°, 21.18±0.20°, 9.06±0.20°, 10.63±0.20°, 11.00±0.20°, 12.85±0.20°, 13.75±0.20°, 15.96±0.20°, 16.19±0.20°, 16.68±0.20°, 16.99±0.20°, 17.51±0.20°, 20.12±0.20°, 20.75±0.20°, 22.71±0.20°, 23.04±0.20°, 24.13±0.20°, 24.55±0.20°, 25.32±0.20°, 25.95±0.20°, 27.31±0.20°, 28.38±0.20°, 28.97±0.20°, 29.62±0.20°, and 34.09±0.20°;
or the crystalline form of the maleate of the compound of formula (I) has an X-ray powder diffraction pattern using Cu Kα radiation as shown in FIG. 9.

**9.** The crystalline form of the compound of formula (I), the salt of the compound of formula (I) or the crystalline form thereof according to claim 8, wherein the crystalline form of the maleate of the compound of formula (I) has a differential scanning calorimetry curve showing an endothermic peak at 160.3±3 °C.

**10.** The crystalline form of the compound of formula (I), the salt of the compound of formula (I) or the crystalline form thereof according to claim 1, wherein the crystalline form of the salt of the compound of formula (I) is the crystalline form of the methanesulfonate, which comprises 3, 4, 5, 6, 7, or 8 diffraction peaks in an X-ray powder diffraction pattern using Cu Kα radiation at 2Θ values selected from the group consisting of: 5.47±0.20°, 6.25±0.20°, 16.11±0.20°, 16.64±0.20°, 18.11±0.20°, 19.73±0.20°, 24.22±0.20°, and 25.12±0.20°;

or the crystalline form of the methanesulfonate of the compound of formula (I) has diffraction peaks in an X-ray powder diffraction pattern using Cu Kα radiation at 2Θ values of 5.47±0.20°, 6.25±0.20°, and 16.11±0.20°;
or the crystalline form of the methanesulfonate of the compound of formula (I) has diffraction peaks in an X-ray powder diffraction pattern using Cu Kα radiation at 2Θ values of 5.47±0.20°, 6.25±0.20°, 16.11±0.20°, 16.64±0.20°, 18.11±0.20°, 19.73±0.20°, 24.22±0.20°, and 25.12±0.20°;
or the crystalline form of the methanesulfonate of the compound of formula (I) has diffraction peaks in an X-ray powder diffraction pattern using Cu Kα radiation at 2Θ values of 5.47±0.20°, 6.00±0.20°, 6.25±0.20°,

8.29±0.20°, 9.62±0.20°, 10.88±0.20°, 12.02±0.20°, 12.46±0.20°, 13.81±0.20°, 16.11±0.20°, 16.39±0.20°, 16.64±0.20°, 18.11±0.20°, 18.72±0.20°, 19.73±0.20°, 20.21±0.20°, 21.08±0.20°, 21.41±0.20°, 22.80±0.20°, 23.45±0.20°, 24.22±0.20°, 24.55±0.20°, 25.12±0.20°, 25.64±0.20°, 26.00±0.20°, 26.99±0.20°, 29.06±0.20°, 29.88±0.20°, and 31.18±0.20°;
or the crystalline form of the methanesulfonate of the compound of formula (I) has an X-ray powder diffraction pattern using Cu Kα radiation as shown in FIG. 11;
or the crystalline form of the methanesulfonate of the compound of formula (I) comprises 3, 4, 5, 6, 7, or 8 diffraction peaks in an X-ray powder diffraction pattern using Cu Kα radiation at 2Θ values selected from the group consisting of: 6.17±0.20°, 8.76±0.20°, 12.16±0.20°, 16.12±0.20°, 17.18±0.20°, 19.23±0.20°, 20.19±0.20°, and 23.03±0.20°;
or the crystalline form of the methanesulfonate of the compound of formula (I) has diffraction peaks in an X-ray powder diffraction pattern using Cu Kα radiation at 2Θ values of 6.17±0.20°, 8.76±0.20°, and 23.03±0.20°;
or the crystalline form of the methanesulfonate of the compound of formula (I) has diffraction peaks in an X-ray powder diffraction pattern using Cu Kα radiation at 2Θ values of 6.17±0.20°, 8.76±0.20°, 12.16±0.20°, 16.12±0.20°, 17.18±0.20°, 19.23±0.20°, 20.19±0.20°, and 23.03±0.20°;
or the crystalline form of the methanesulfonate of the compound of formula (I) has diffraction peaks in an X-ray powder diffraction pattern using Cu Kα radiation at 2Θ values of 6.17±0.20°, 8.76±0.20°, 12.16±0.20°, 12.37±0.20°, 14.53±0.20°, 15.46±0.20°, 16.12±0.20°, 17.18±0.20°, 17.40±0.20°, 18.30±0.20°, 18.78±0.20°, 19.23±0.20°, 19.71±0.20°, 20.19±0.20°, 20.74±0.20°, 21.05±0.20°, 22.19±0.20°, and 23.03±0.20°;
or the crystalline form of the methanesulfonate of the compound of formula (I) has an X-ray powder diffraction pattern using Cu Kα radiation as shown in FIG. 12.

**11.** The crystalline form of the compound of formula (I), the salt of the compound of formula (I) or the crystalline form thereof according to claim 1, wherein the crystalline form of the salt of the compound of formula (I) is the crystalline form of the hydrobromide, which comprises 3, 4, 5, 6, 7, or 8 diffraction peaks in an X-ray powder diffraction pattern using Cu Kα radiation at 2Θ values selected from the group consisting of: 3.12±0.20°, 5.96±0.20°, 7.59±0.20°, 16.49±0.20°, 17.62±0.20°, 23.84±0.20°, 24.54±0.20°, and 30.30±0.20°;

or the crystalline form of the hydrobromide of the compound of formula (I) has diffraction peaks in an X-ray powder diffraction pattern using Cu Kα radiation at 2Θ values of 7.59±0.20°, 17.62±0.20°, and 23.84±0.20°;
or the crystalline form of the hydrobromide of the compound of formula (I) has diffraction peaks in an X-ray powder diffraction pattern using Cu Kα radiation at 2Θ values of 3.12±0.20°, 5.96±0.20°, 7.59±0.20°, 16.49±0.20°, 17.62±0.20°, 23.84±0.20°, 24.54±0.20°, and 30.30±0.20°;
or the crystalline form of the hydrobromide of the compound of formula (I) has diffraction peaks in an X-ray powder diffraction pattern using Cu Kα radiation at 2Θ values of 3.12±0.20°, 5.96±0.20°, 7.59±0.20°, 9.76±0.20°, 11.11±0.20°, 12.78±0.20°, 16.49±0.20°, 17.62±0.20°, 18.44±0.20°, 19.57±0.20°, 20.69±0.20°, 21.16±0.20°, 22.16±0.20°, 22.77±0.20°, 23.84±0.20°, 24.31±0.20°, 24.54±0.20°, 25.15±0.20°, 26.07±0.20°, 27.31±0.20°, 27.83±0.20°, 28.91±0.20°, 30.30±0.20°, and 31.74±0.20°;
or the crystalline form of the hydrobromide of the compound of formula (I) has an X-ray powder diffraction pattern using Cu Kα radiation as shown in FIG 13.

**12.** The crystalline form of the compound of formula (I), the salt of the compound of formula (I) or the crystalline form thereof according to any of claims 1-11, wherein

the salt of the compound of formula (I) is a hydrochloride, and a molar ratio of the compound of formula (I) to hydrochloric acid is 1:1;
or the salt of the compound of formula (I) is a fumarate, and a molar ratio of the compound of formula (I) to fumaric acid is 1:1;
or the salt of the compound of formula (I) is a maleate, and a molar ratio of the compound of formula (I) to maleic acid is 1:1;
or the salt of the compound of formula (I) is a methanesulfonate, and a molar ratio of the compound of formula (I) to methanesulfonic acid is 1:2;
or the salt of the compound of formula (I) is a hydrobromide, and a molar ratio of the compound of formula (I) to hydrobromic acid is 1:1.

**13.** A crystalline composition, comprising the crystalline form according to any of claims 1-12, wherein the crystalline form makes up 50% or more, preferably 80% or more, more preferably 90% or more, and most preferably 95% or

more, by weight, of the crystalline composition.

**14.** A pharmaceutical composition, comprising a therapeutically or prophylactically effective amount of the crystalline form of the compound of formula (I), the salt of the compound of formula (I) or the crystalline form thereof according to any of claims 1-12, or the crystalline composition according to claim 13.

**15.** The crystalline form of the compound of formula (I), the salt of the compound of formula (I) or the crystalline form thereof according to any of claims 1-12, the crystalline composition according to claim 13, or the pharmaceutical composition according to claim 14 for use in treating or preventing an ER-related disease, wherein preferably, the ER-related disease is breast cancer; more preferably, the ER-related disease is ER-positive breast cancer.

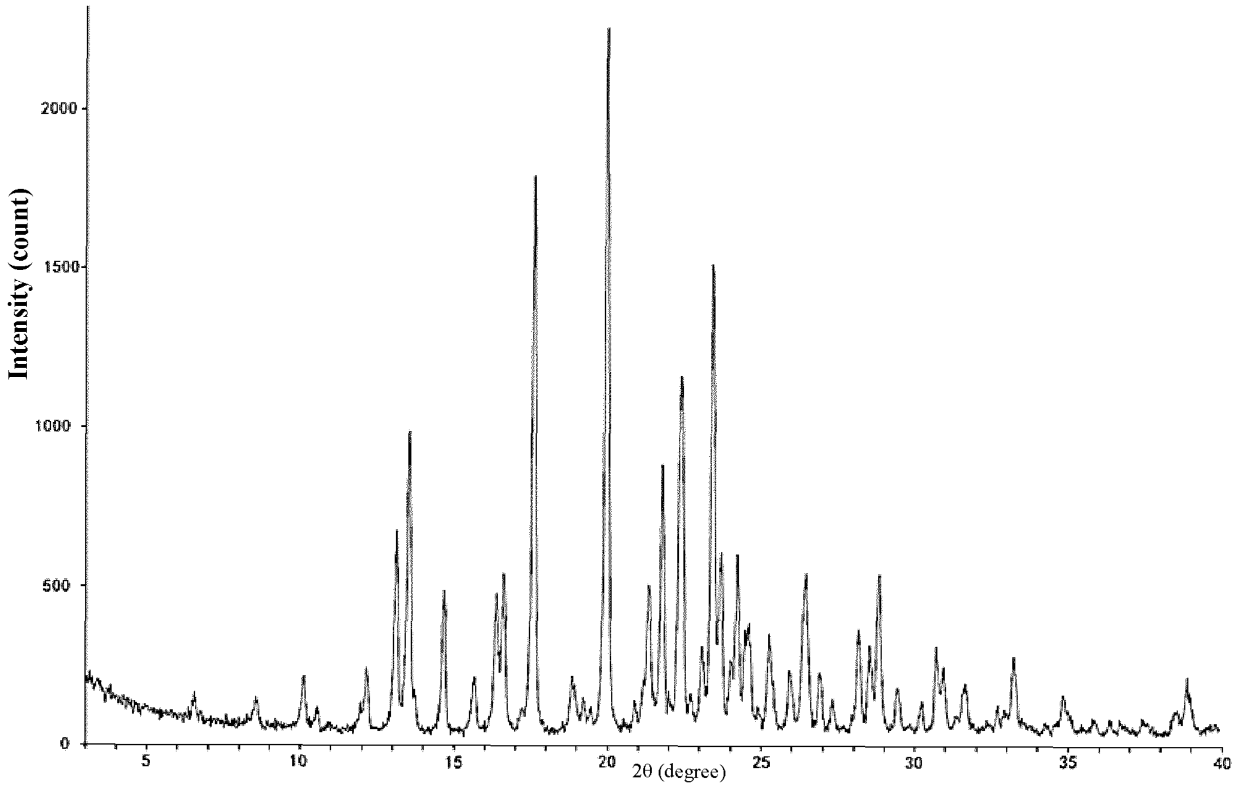
Intensity (count)
2000
1500
1000
500
0
5
10
15
20
25
30
35
40
2θ (degree)

FIG. 1

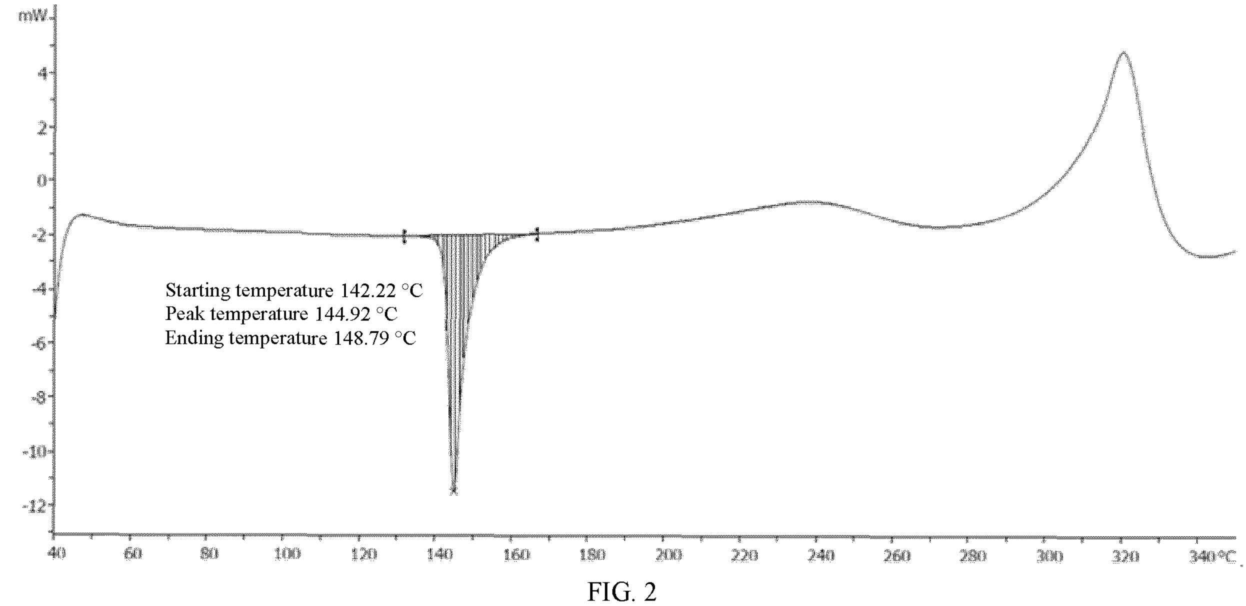
mW
4
2
0
-2
-4
-6
-8
-10
-12
Starting temperature 142.22 °C
Peak temperature 144.92 °C
Ending temperature 148.79 °C
40
60
80
100
120
140
160
180
200
220
240
260
280
300
320
340°C

FIG. 2

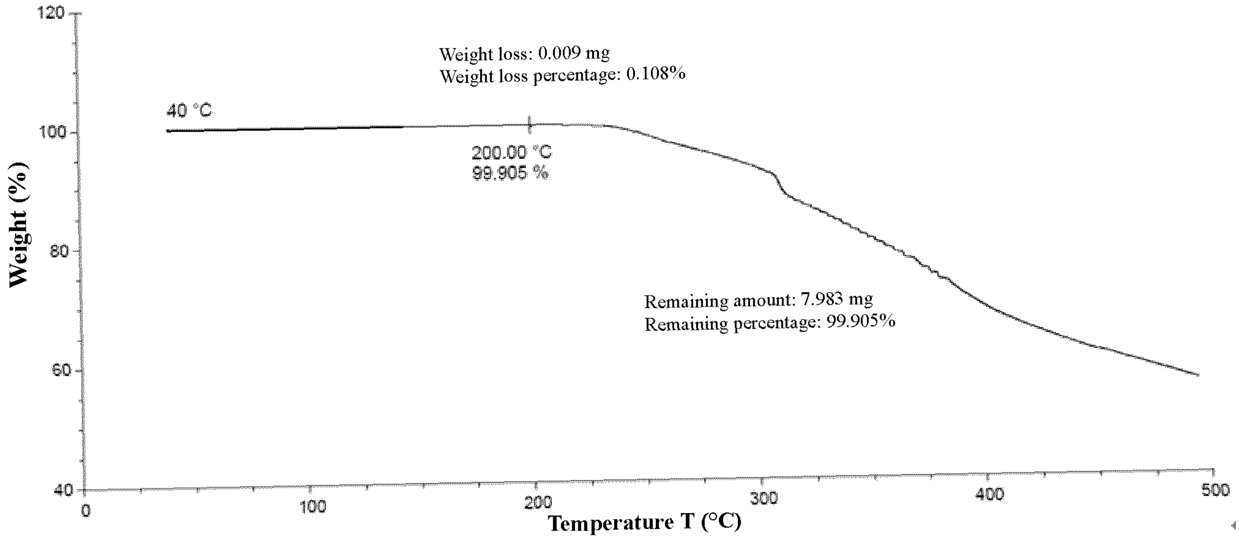
Weight loss: 0.009 mg
Weight loss percentage: 0.108%
40 °C
200.00 °C
99.905 %
Remaining amount: 7.983 mg
Remaining percentage: 99.905%
Weight (%)
Temperature T (°C)
120
100
80
60
40
0
100
200
300
400
500

FIG. 3

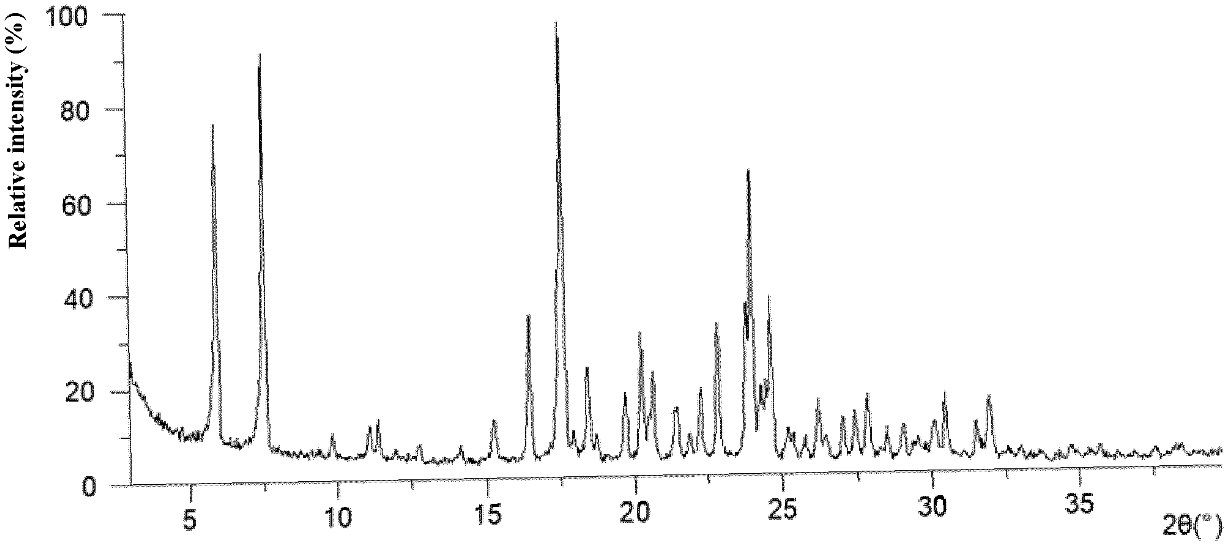
Relative intensity (%)
100
80
60
40
20
0
5
10
15
20
25
30
35
2θ(°)

FIG. 4

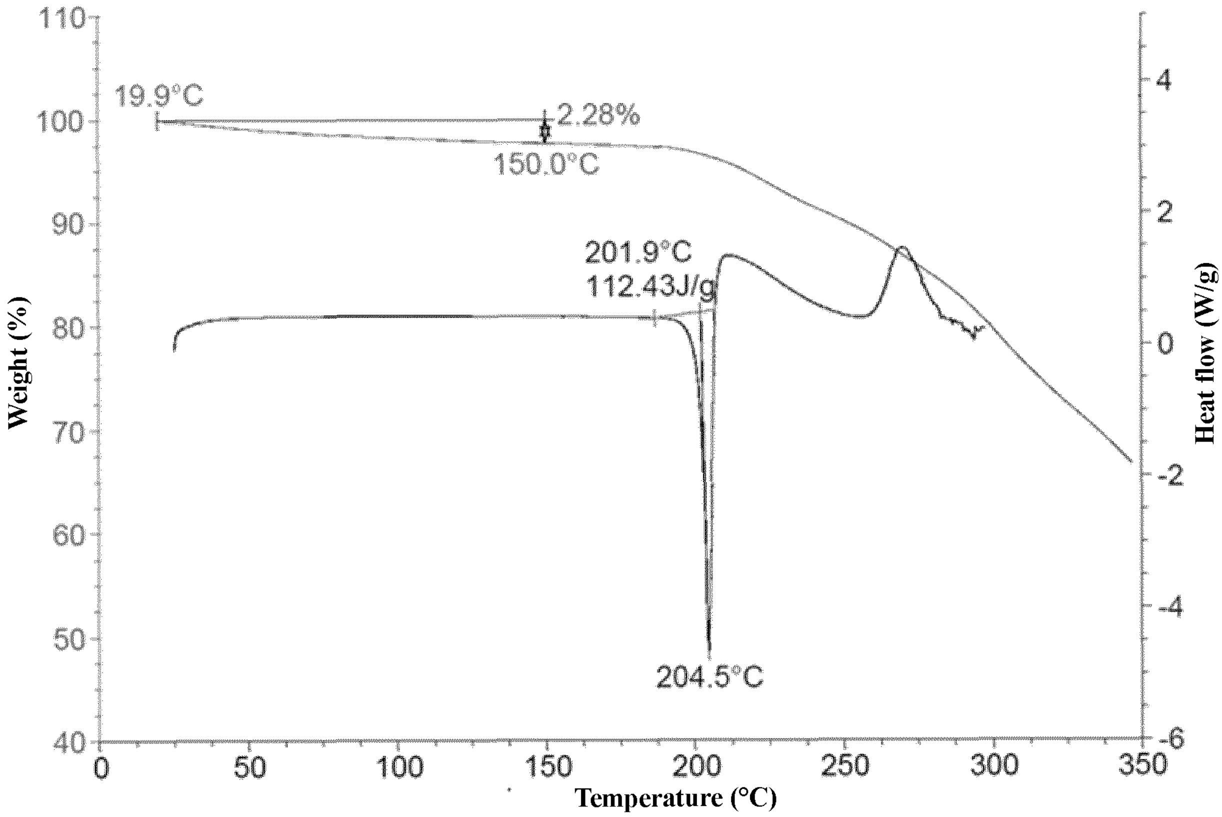
110
100
90
80
70
60
50
40
0
50
100
150
200
250
300
350
4
2
0
-2
-4
-6
19.9°C
2.28%
150.0°C
201.9°C
112.43J/g
204.5°C
Weight (%)
Heat flow (W/g)
Temperature (°C)
FIG. 5

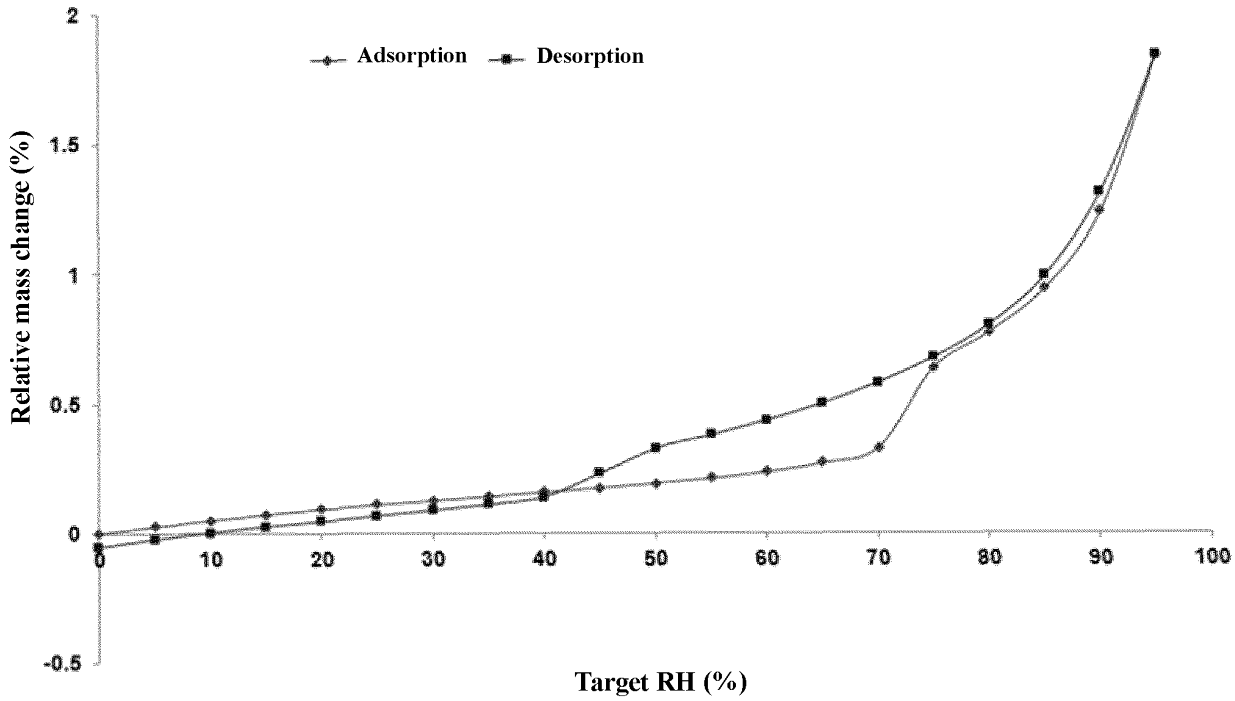
Adsorption
Desorption
2
1.5
1
0.5
0
-0.5
0
10
20
30
40
50
60
70
80
90
100
Relative mass change (%)
Target RH (%)
FIG. 6

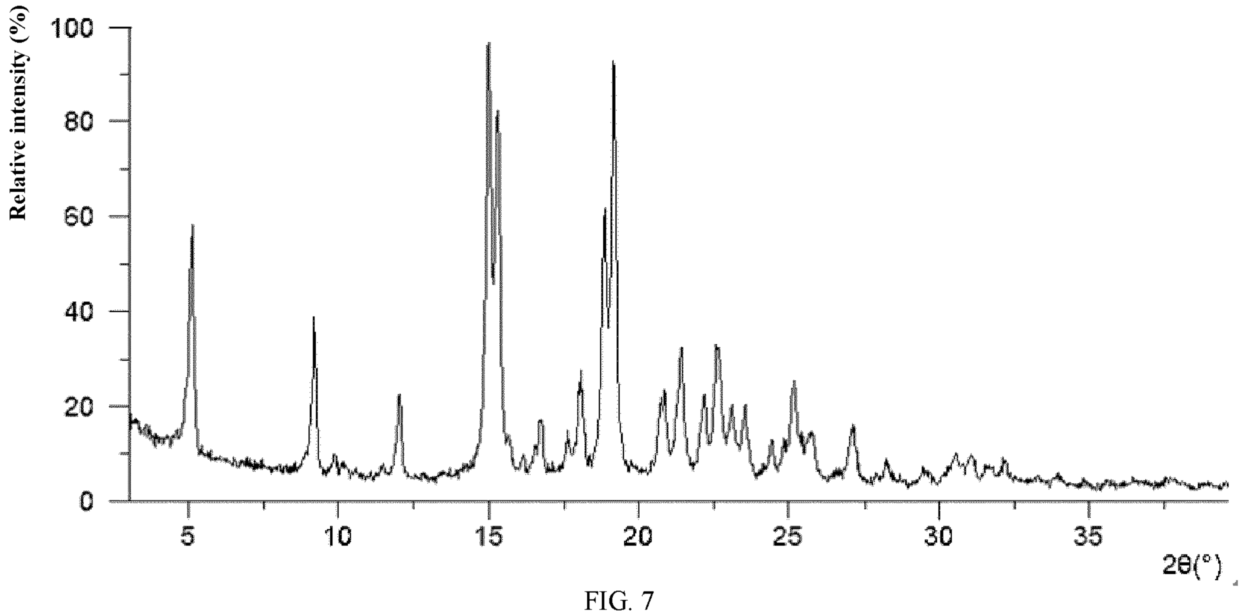
Relative intensity (%)
100
80
60
40
20
0
5
10
15
20
25
30
35
2θ(°)

FIG. 7

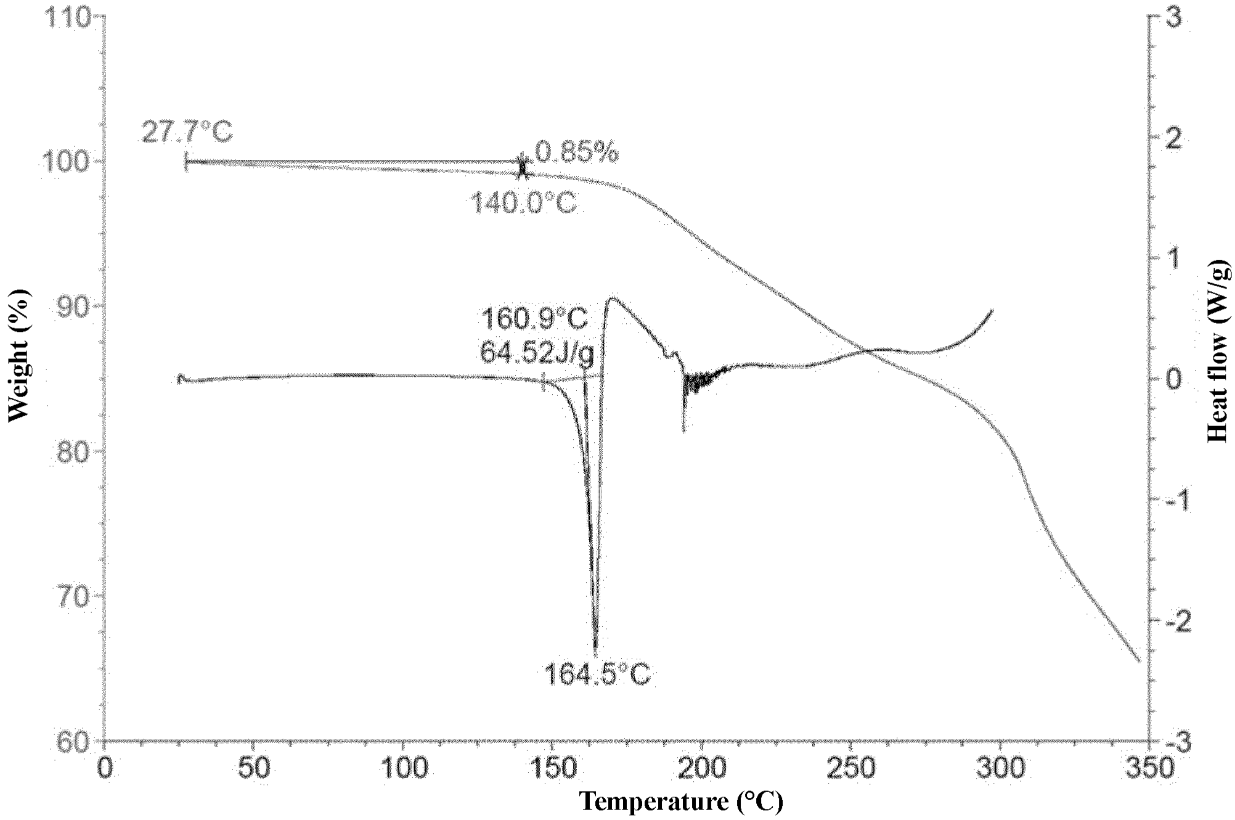
110
100
90
80
70
60
Weight (%)
3
2
1
0
-1
-2
-3
Heat flow (W/g)
27.7°C
0.85%
140.0°C
160.9°C
64.52J/g
164.5°C
0
50
100
150
200
250
300
350
Temperature (°C)

FIG. 8

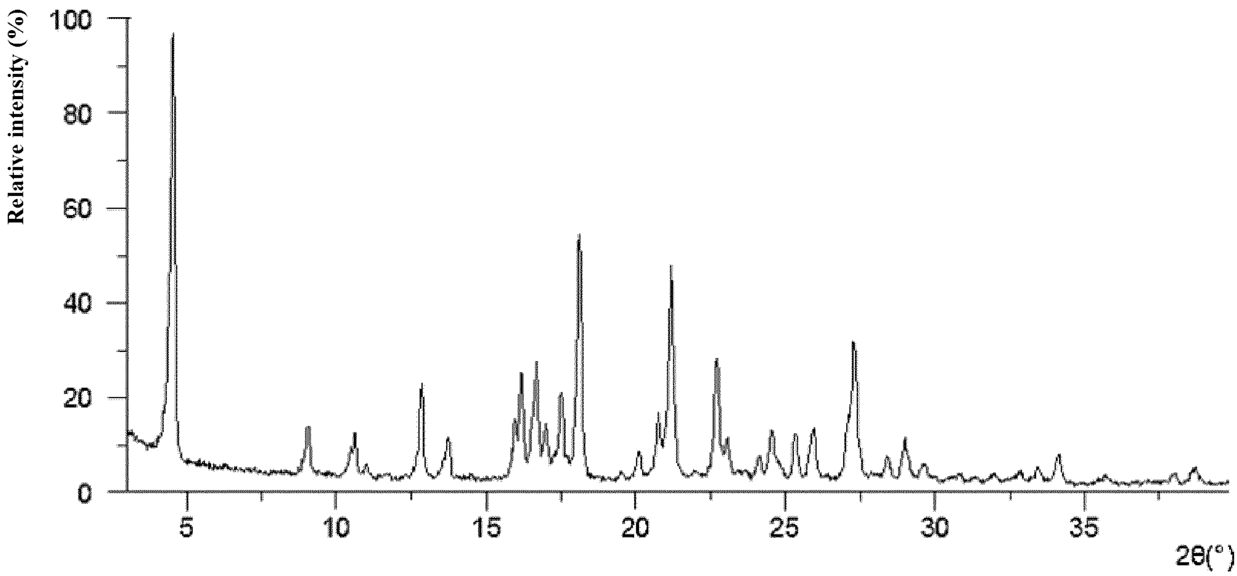
Relative intensity (%)
100
80
60
40
20
0
5
10
15
20
25
30
35
2θ(°)

FIG. 9

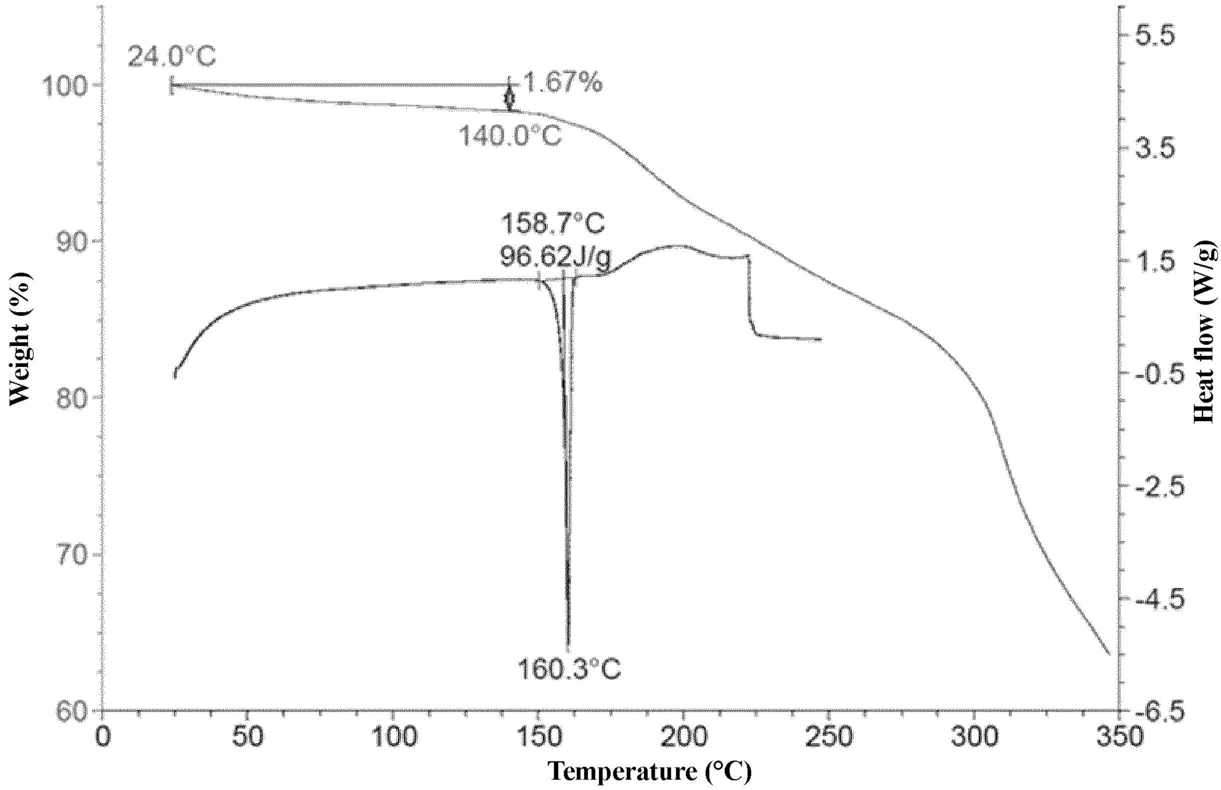
24.0°C
1.67%
140.0°C
158.7°C
96.62J/g
160.3°C
Weight (%)
Heat flow (W/g)
100
90
80
70
60
5.5
3.5
1.5
-0.5
-2.5
-4.5
-6.5
0
50
100
150
200
250
300
350
Temperature (°C)

FIG. 10

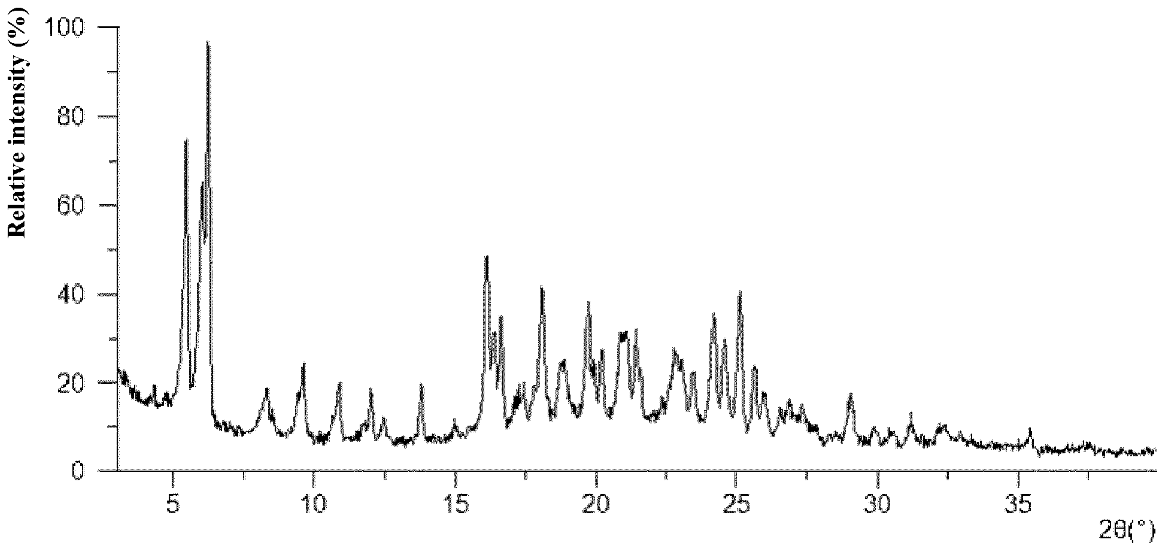


FIG. 11

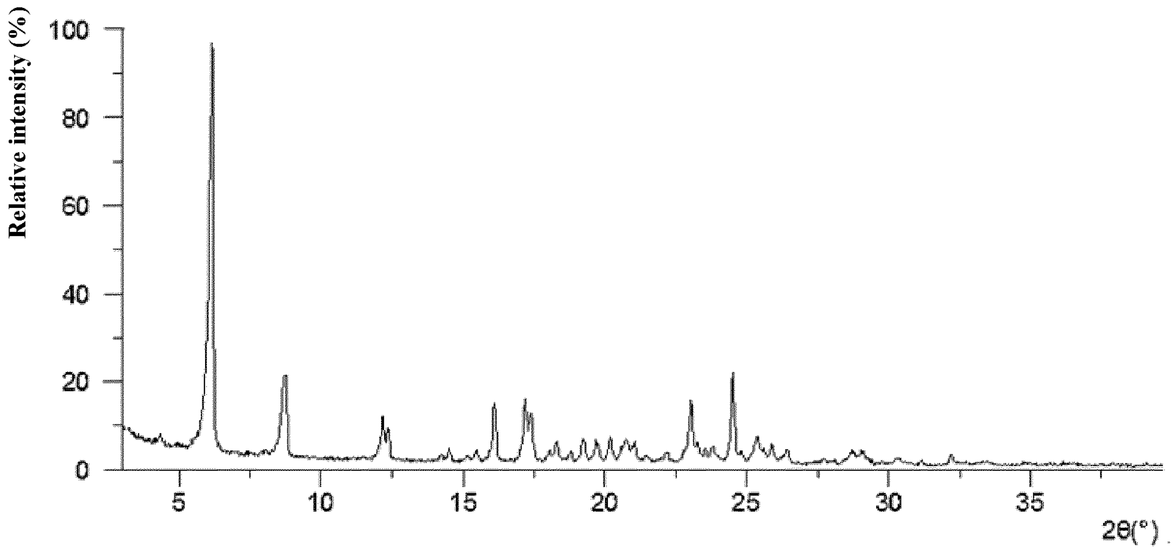


FIG. 12

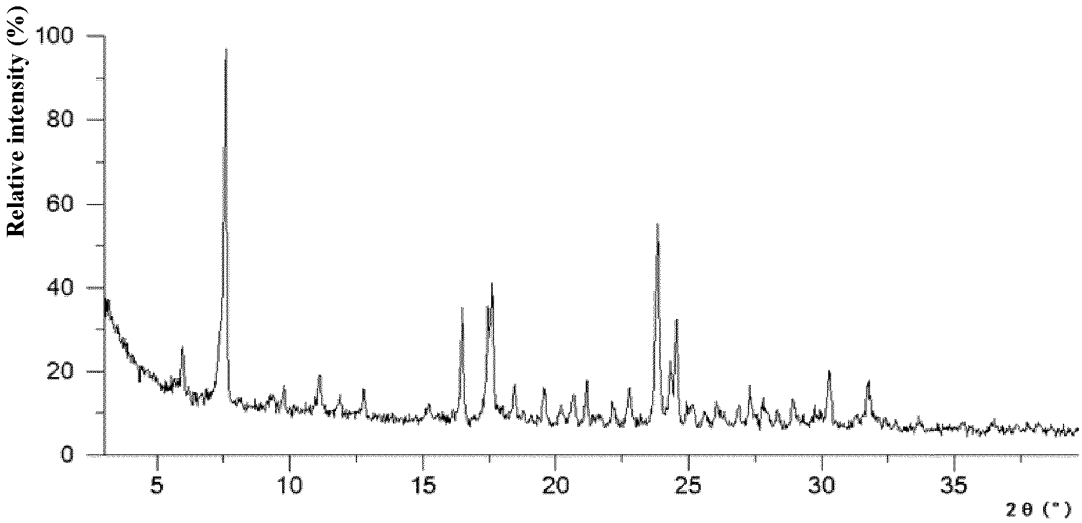


FIG. 13

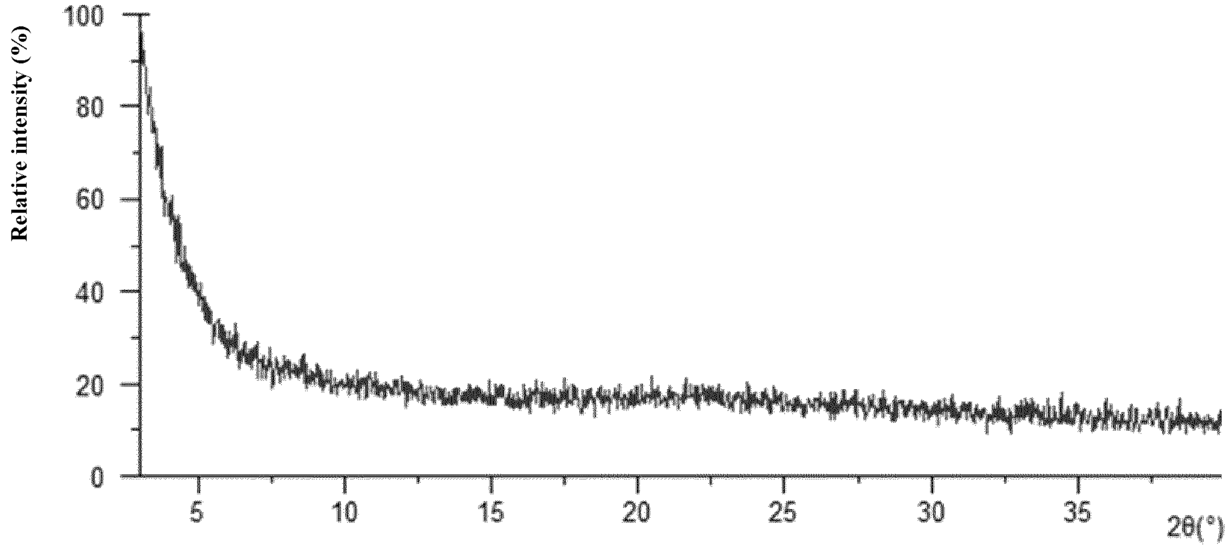
Relative intensity (%)
100
80
60
40
20
0
5
10
15
20
25
30
35
2θ(°)

FIG. 14

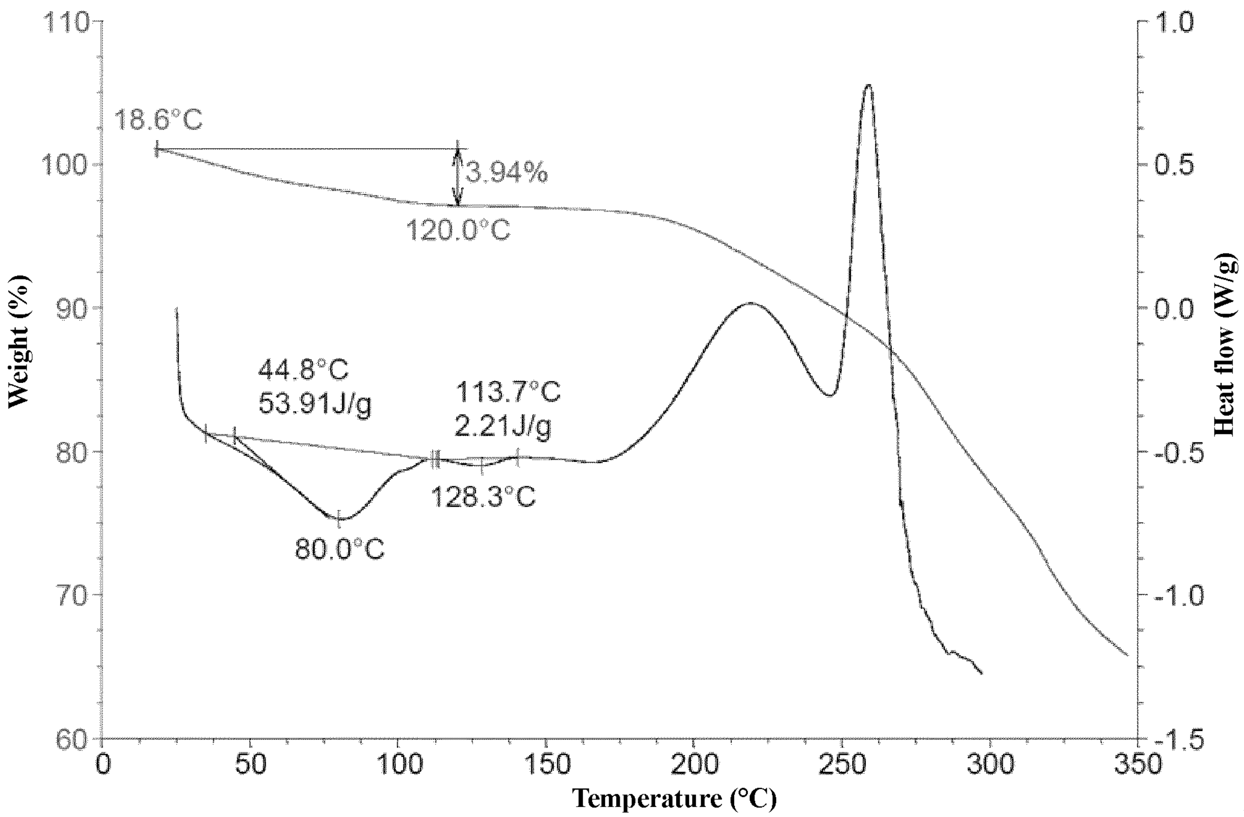
110
100
90
80
70
60
Weight (%)
1.0
0.5
0.0
-0.5
-1.0
-1.5
Heat flow (W/g)
18.6°C
3.94%
120.0°C
44.8°C
53.91J/g
113.7°C
2.21J/g
128.3°C
80.0°C
0
50
100
150
200
250
300
350
Temperature (°C)

FIG. 15

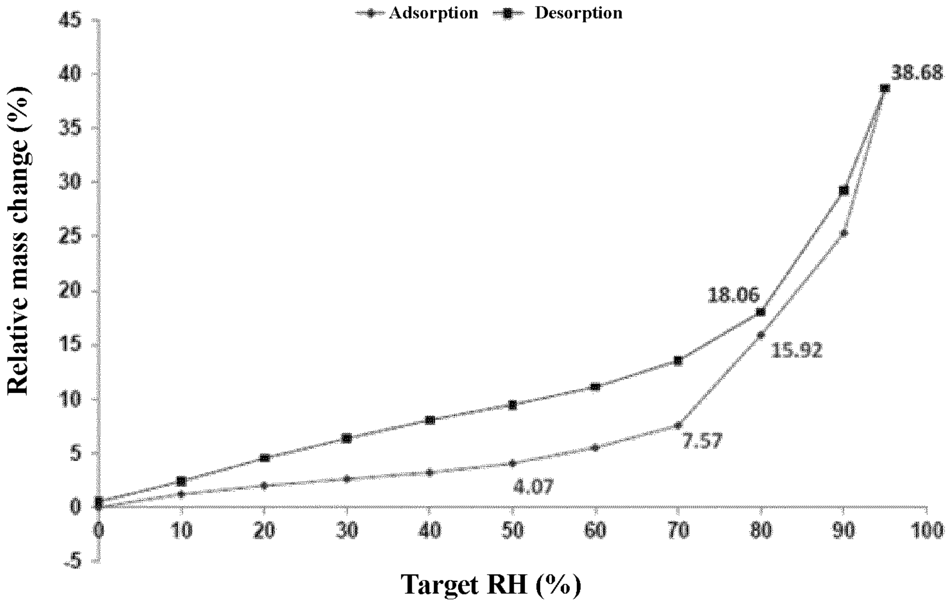
Adsorption
Desorption
Relative mass change (%)
45
40
35
30
25
20
15
10
5
0
-5
0
10
20
30
40
50
60
70
80
90
100
38.68
18.06
15.92
7.57
4.07
Target RH (%)

FIG. 16

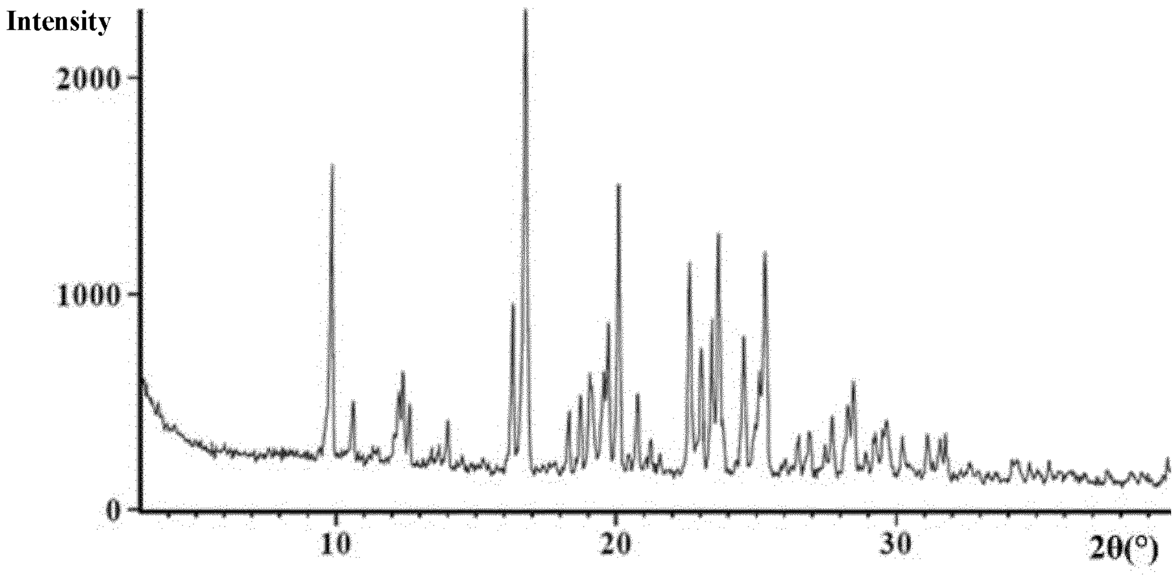
Intensity
2000
1000
0
10
20
30
2θ(°)

FIG. 17

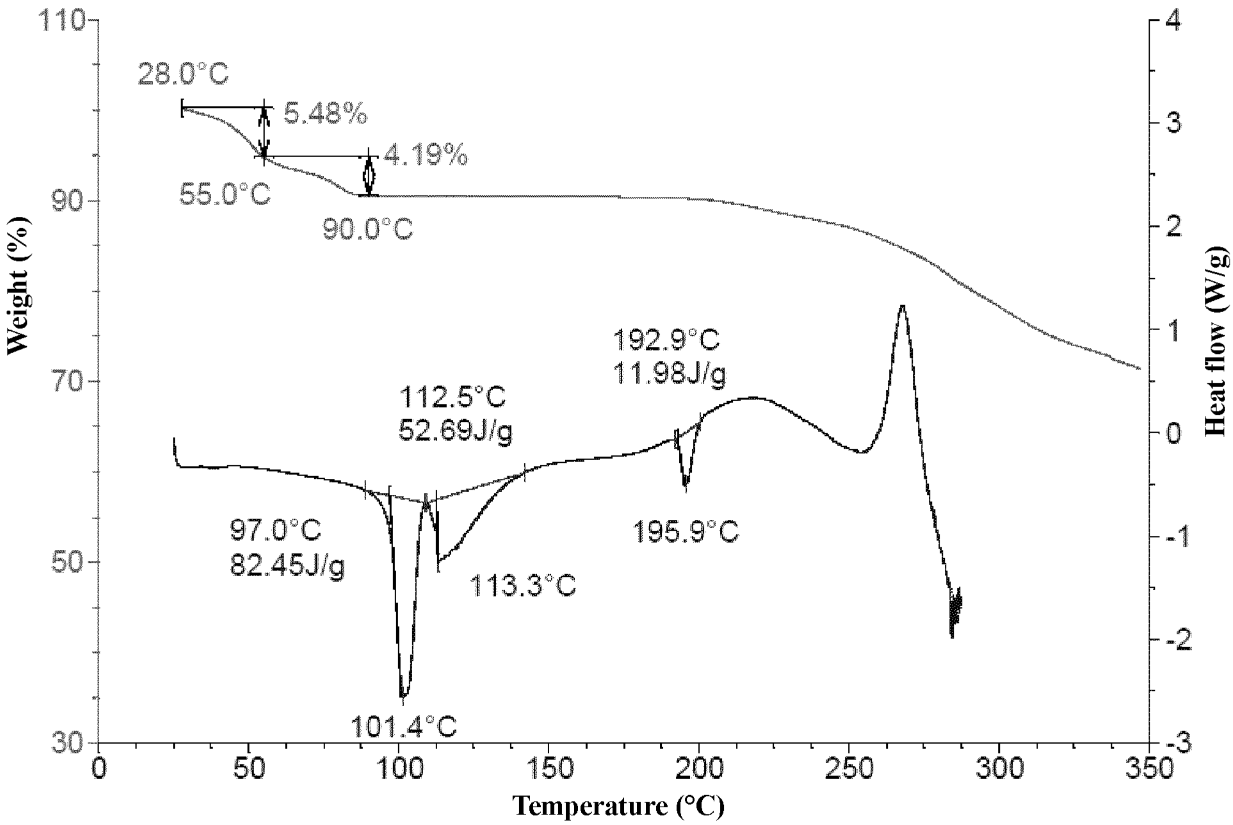
110
90
70
50
30
Weight (%)
4
3
2
1
0
-1
-2
-3
Heat flow (W/g)
28.0°C
5.48%
55.0°C
4.19%
90.0°C
192.9°C
11.98J/g
112.5°C
52.69J/g
97.0°C
82.45J/g
113.3°C
195.9°C
101.4°C
0
50
100
150
200
250
300
350
Temperature (°C)
FIG. 18

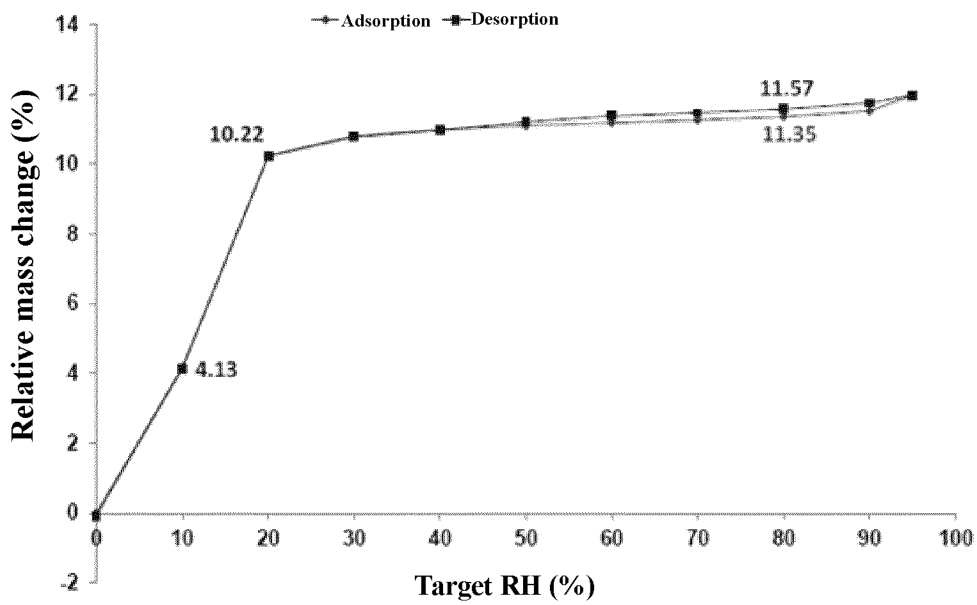
Adsorption
Desorption
14
12
10
8
6
4
2
0
-2
Relative mass change (%)
11.57
10.22
11.35
4.13
0
10
20
30
40
50
60
70
80
90
100
Target RH (%)
FIG. 19

## INTERNATIONAL SEARCH REPORT

International application No.
**PCT/CN2021/143129**

**A. CLASSIFICATION OF SUBJECT MATTER**

C07D 401/10(2006.01)i; A61K 31/4439(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN(CAPLUS, REGISTRY): 正大天晴药业, 2439210-66-7, 2439210-94-1

**C. DOCUMENTS CONSIDERED TO BE RELEVANT**

| Category* | Citation of document, with indication, where appropriate, of the relevant passages | Relevant to claim No. |
|---|---|---|
| X | WO 2020125640 A1 (CHIA TAI TIANQING PHARMACEUTICAL GROUP CO., LTD. et al.) 25 June 2020 (2020-06-25)<br>abstract, and embodiment 3 | 1-15 |
| A | CN 110267940 A (EISAI R&D MANAGEMENT CO., LTD.) 20 September 2019 (2019-09-20)<br>entire document | 1-15 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2022** | **01 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/143129**

| Patent document cited in search report | Publication date (day/month/year) | Patent family member(s) | Publication date (day/month/year) |
|---|---|---|---|
| WO 2020125640 A1 | 25 June 2020 | PH 12021551437 A1 | 06 December 2021 |
| | | SG CN 11202106401U A | 29 July 2021 |
| | | JP 2022513942 A | 09 February 2022 |
| | | KR 20210105384 A | 26 August 2021 |
| | | CA 3122621 A1 | 25 June 2020 |
| | | EP 3889133 A1 | 06 October 2021 |
| | | US 2022033376 A1 | 03 February 2022 |
| | | BR 112021011728 A2 | 31 August 2021 |
| | | CN 113166055 A | 23 July 2021 |
| | | AU 2019400398 A1 | 15 July 2021 |
| CN 110267940 A | 20 September 2019 | MA 46896 A | 02 October 2019 |
| | | EP 3544956 A1 | 02 October 2019 |
| | | JP 2019535781 A | 12 December 2019 |
| | | US 2019337921 A1 | 07 November 2019 |
| | | WO 2018098251 A1 | 31 May 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011613790
- WO 2020125640 A